# EUROPEAN PATENT SPECIFICATION

(11) **EP 3 348 193 B1**
(45) Date of publication and mention of the grant of the patent: **01.06.2022**
(21) Application number: 16843909.9
(22) Date of filing: 29.08.2016
(51) Int. Cl.: A61B 6/00, A61B 6/10

(54) **CONVEYANCE ASSISTANCE METHOD AND CONVEYANCE ASSISTANCE DEVICE FOR RADIATION EMITTING DEVICE, AND RADIOLOGICAL IMAGING DEVICE**
FÖRDERUNTERSTÜTZUNGSVERFAHREN UND FÖRDERUNTERSTÜTZUNGSVORRICHTUNG FÜR STRAHLUNGSEMISSIONSVORRICHTUNG UND RADIOLOGISCHE BILDGEBUNGSVORRICHTUNG
PROCÉDÉ ET DISPOSITIF D'ASSISTANCE AU DÉPLACEMENT POUR DISPOSITIF D'ÉMISSION DE RAYONNEMENT, ET DISPOSITIF D'IMAGERIE RADIOLOGIQUE

(30) Priority: 08.09.2015 JP 2015176637
(43) Date of publication of application: 18.07.2018
(73) Proprietor: FUJIFILM Corporation, Tokyo 106-8620 (JP)
(72) Inventor: TSUJI, Tetsuya, Ashigarakami-gun Kanagawa 258-8538 (JP); NAKATSUGAWA, Haruyasu, Ashigarakami-gun Kanagawa 258-8538 (JP); OHKUBO, Takeshi, Ashigarakami-gun Kanagawa 258-8538 (JP)
(74) Representative: Klunker IP Patentanwälte PartG mbB
(86) International application number: PCT/JP2016/003917
(87) International publication number: WO 2017/043040

(56) References cited:
- EP-A1- 2 380 496
- DE-A1-102011 005 439
- JP-A- 2003 310 591
- JP-A- 2004 041 698
- JP-A- 2006 141 669
- JP-A- 2009 178 361
- JP-A- 2011 229 900
- JP-A- 2014 533 188
- US-A1- 2014 241 504
- US-A1- 2014 372 037
- US-A1- 2015 134 145

## Description

### Technical Field

The present invention relates to a transport assisting method and transport assisting device for assisting the transport of a radiation-irradiation device that irradiates a subject with radiation in a case in which the radiation image of the subject is to be acquired.

Further, the present invention relates to a radiographic imaging apparatus using the transport assisting device.

### Background Art

In the past, a portable radiation-irradiation device, on which only a minimum number of components for radiation irradiation, such as a radiation source and an electrical circuit, are mounted and which can be operated while being held with hands manually by an operator, has been proposed as disclosed in, for example, "Toshiba Medical Supply Co., Ltd., X-ray equipment IPF-21, [online], [Search on July 30, 1999], Internet URL:http://www.toshiba-iryouyouhin.co.jp/tmeds/xrays/ipf21.html". Since this kind of portable radiation-irradiation device is reduced in weight so that an operator can hold and operate the radiation-irradiation device manually with hands, the radiation-irradiation device is advantageous for the imaging of a subject in various directions.

A radiation detector (so-called "Flat Panel Detector"), which records a radiation image representing a subject by being irradiated with radiation transmitted through the subject, is generally used in a case in which the radiation image of the subject is to be taken by this kind of radiographic imaging apparatus. A cassette-type radiation detector having a structure in which an image detection unit and a control unit, such as a battery for drive and an electrical circuit relating to drive, are received in a housing is well known as the radiation detector. Further, in a case in which such a radiation detector is disposed at a position facing the radiation-irradiation device with a subject interposed therebetween and the radiation-irradiation device is driven in this state, the radiation detector is irradiated with radiation transmitted through the subject. Accordingly, a radiation image represented by the radiation transmitted through the subject is acquired.

The portable radiation-irradiation device can be held and operated with hands by an operator. However, a radiation-irradiation device, which includes a holding unit holding a radiation source unit including a radiation source, is proposed to prevent shaking and to prevent operator's hands or the like from being exposed to radiation. "Toshiba Medical Supply Co., Ltd., X-ray equipment IPF-21, [online], [Search on July 30, 1999], Internet URL:http://www.toshibairyouyouhin.co.jp/tmeds/xrays/ipf21.html" also discloses an example of such a holding unit, and particularly, a holding unit that includes wheel parts provided at lower portions of holding legs and can travel.

The radiation-irradiation device including the holding unit basically includes: a leg unit that is adapted to be capable of traveling using wheels; a body unit that receives a control unit including a battery for the drive of a radiation source, an electrical circuit relating to the drive of the radiation source, and the like and is held on the leg unit; and an arm unit that is connected to the body unit. The radiation source unit is mounted on the distal end of the arm unit.

In a case in which the radiation-irradiation device, which is adapted to be capable of traveling as described above, is used to take a radiation image, the radiation-irradiation device is transported to an imaging site by a transport force of an operator (user). However, the collision or interference between the radiation-irradiation device and a transport path or obstacles which are present near the transport path, should be naturally avoided during the transport of the radiation-irradiation device. JP2015-006333A discloses a device that specifies the positions of obstacles present in an examination room on the basis of a plurality of images relating to the inside of the examination room and prevents the collision by limiting the moving range of a radiation source holding unit on the basis of the positions of the obstacles and the like.

Further, JP2014-168690A discloses a device that acquires information about a relative positional relationship between a radiation source and a radiation detector and guides and controls the movement position of a radiation-irradiation device capable of traveling on the basis of the information about the positional relationship.

US 2014/241504 A1 discloses a transport assisting method, but this prior art method does not take into account the position of an obstacle. Rather, proximity sensors and a camera are provided and the images are sent to a screen so that the user can see what is in front of the apparatus.

### SUMMARY OF THE INVENTION

### Technical Problem

However, in a case in which the appropriate position of the radiation-irradiation device holding the radiation source, which is obtained at the time of imaging, is determined on the basis of only the relative positional relationship between the radiation source and the radiation detector as disclosed in JP2014-168690A, an obstacle is present close to the radiation-irradiation device disposed at the appropriate position. For this reason, there is a problem that it is not possible to perform work for taking a radiation image or it is difficult to perform work for taking a radiation image. The device disclosed in JP2015-006333A is the same in terms of being incapable of preventing the generation of the above-mentioned problem.

The invention has been made in consideration of the above-mentioned circumstances, and an object of the invention is to provide a transport assisting method for a radiation-irradiation device that can assist the setting of the position of a radiation-irradiation device holding a radiation source, which is obtained at the time of imaging, to an appropriate position at which work relating to imaging can be performed.

Further, an object of the invention is to provide a transport assisting device that can perform the above-mentioned transport assisting method for a radiation-irradiation device.

Furthermore, an object of the invention is to provide a radiographic imaging apparatus that can perform the above-mentioned transport assisting method for a radiation-irradiation device.

### Solution to Problem

A transport assisting method for a radiation-irradiation device according to the invention comprises the features of claim 1.

Here, "is capable of being transported by the user" includes not only a case in which the user can transport the radiation-irradiation device by only one's own transport force but also a case in which the radiation-irradiation device includes a power unit, such as a motor, and can be transported by only power while a user operates the power unit or can be transported while the user uses power as an assisting force.

Further, the setting of the target position, the detection of the current position, and the detection of the position of the obstacle are not limited to a case in which the setting of the target position, the detection of the current position, and the detection of the position of the obstacle are performed in this order, and may be performed in any other arbitrary order.

Furthermore, the setting of the target position does not necessarily need to be performed in preference to all other processing. For example, a distance between the radiation-irradiation device and the radiation image recording medium may be detected by the radiation-irradiation device on the basis of, for example, the strength of a radio signal transmitted from the radiation image recording medium before the setting of the target position, the setting of the target position may be started in a case in which this distance is shortened to a distance determined in advance, and the notifying may be performed subsequent to the start of the setting of the target position. Alternatively, in a case in which an emergency treatment room includes a plurality of rooms, for example, a room A, a room B, a room C, ..., and the radiation-irradiation device is currently used in the room A and is to be used in the room B next, the setting of the target position may be started under conditions where the radiation-irradiation device enters the room B and/or leaves the room A and the notifying may be performed subsequent to the start of the setting of the target position.

Preferred embodiments are defined by the dependent claims. Here, the "predetermined distance" is a distance determined in advance, and does not mean a distance having a specific value.

In the transport assisting method defined by claim 12, a dedicated element may be used as this display means, or other display means for performing input/output for the control of the radiation-irradiation device or for displaying an image used to confirm a radiation-irradiation field may be used as this display means.

In the transport assisting method defined by claim 17 "Notifying the transport direction of the radiation irradiating device with respect to the transport direction at a point of time when the notification is performed" is to notify the user of how to change the transport direction with respect to the transport direction of the radiation-irradiation device at a point of time when the notifying is performed or to notify the user of whether or not to change the transport direction, and more specifically means "go straight", "turn right", or "turn left".

A transport assisting device for a radiation-irradiation device according to the invention is defined by the features of claim 20.

Further, a radiographic imaging apparatus according to the invention includes the claimed transport assisting device.

### Advantageous Effects of Invention

The transport assisting method for a radiation-irradiation device of the invention includes: setting a target position of the radiation-irradiation device where the radiation source is capable of being disposed at a radiation-irradiation position where a radiation image is capable of being taken with respect to the radiation image recording medium in a movable range where the position of the radiation source is capable of being changed by the radiation source holding unit and a work space is capable of being secured around the radiation-irradiation device; detecting a current position of the radiation-irradiation device; detecting a position of an obstacle that obstructs the transport of the radiation-irradiation device; and notifying the user of the transport assistance of the radiation-irradiation device to the target position on the basis of the target position, the current position, and the position of the obstacle. Accordingly, it is possible to assist the setting of the position of a radiation-irradiation device holding a radiation source, which is obtained at the time of imaging to an appropriate position around which work can also be performed.

### BRIEF DESCRIPTION OF THE DRAWINGS

Fig. 1 is a perspective view showing the shape of the entire radiation-irradiation device that performs a transport assisting method according to an embodiment of the invention.
Fig. 2 is a diagram showing a state in which the radiation-irradiation device is in use.
Fig. 3 is a diagram of a part of the radiation-irradiation device viewed in the direction of an arrow A of Fig. 2.
Fig. 4 is a perspective view showing a state in which the radiation-irradiation device is in use.
Fig. 5 is a perspective view showing the appearance of a radiation detector viewed from a front surface that is a radiation-irradiation side.
Fig. 6 is a block diagram showing the schematic configuration of a transport assisting device according to an embodiment of the invention.
Fig. 7 is a flow chart showing the flow of a part of processing of the transport assisting method according to the embodiment of the invention.
Fig. 8 is a flow chart showing the flow of a part of processing of the transport assisting method according to the embodiment of the invention.
Fig. 9 is a schematic diagram showing one example of a display image that is used to assist the transport of the radiation-irradiation device.
Fig. 10 is a schematic diagram showing another example of the display image that is used to assist the transport of the radiation-irradiation device.
Fig. 11 is a diagram illustrating the transport assisting method according to the embodiment of the invention.
Fig. 12 is a diagram illustrating the transport assisting method according to the embodiment of the invention.
Fig. 13 is a diagram illustrating the transport assisting method according to the embodiment of the invention.
Fig. 14 is a diagram illustrating the transport assisting method according to the embodiment of the invention.
Fig. 15 is a schematic diagram showing another example of a monitor display image of the radiation-irradiation device.
Fig. 16 is a schematic diagram showing still another example of the monitor display image of the radiation-irradiation device.
Fig. 17 is a schematic diagram showing yet another example of the monitor display image of the radiation-irradiation device.
Fig. 18 is a flow chart showing the flow of a part of processing of the transport assisting method according to the embodiment of the invention.
Fig. 19 is a diagram illustrating an example of a target position-setting timing of the radiation-irradiation device.
Fig. 20 is a diagram illustrating another example of the target position-setting timing of the radiation-irradiation device.
Fig. 21 is a schematic diagram showing still another example of the monitor display image of the radiation-irradiation device.
Fig. 22 is a perspective view showing another example of a wheel part that is applied to the radiation-irradiation device.

### DESCRIPTION OF EMBODIMENTS

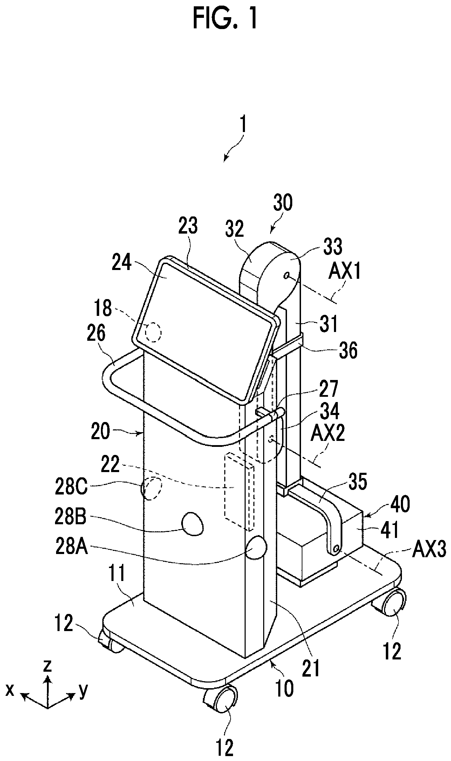

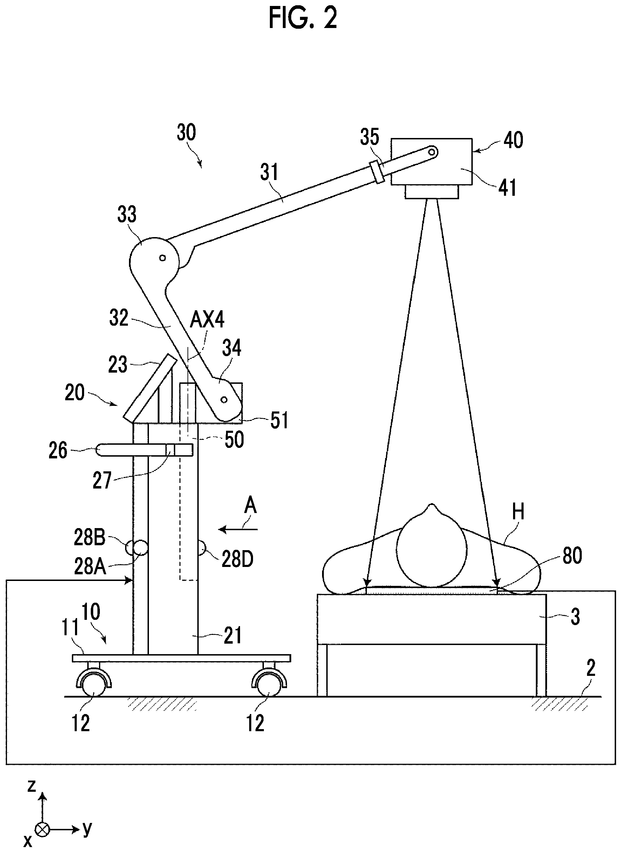

Embodiments of the invention will be described below with reference to the drawings. Fig. 1 is a perspective view showing the shape of the entire radiation-irradiation device 1 that performs a transport assisting method according to an embodiment of the invention and is not in use, and Fig. 2 is a side view showing a state in which the radiation-irradiation device 1 is in use. In the following description, the upper side and the lower side in a vertical direction in a state in which the radiation-irradiation device 1 is placed on a device-placement surface, such as the floor of, for example, a medical facility, are referred to as "upper" and "lower", and a direction perpendicular to the vertical direction in the same state as the state is referred to as a "horizontal" direction. Further, the vertical direction is defined as a z direction, a lateral direction of Fig. 2 is defined as a y direction, and a direction perpendicular to the plane of Fig. 2 is defined as an x direction in the following description. Furthermore, since the front side of the radiation-irradiation device 1 corresponds to +y direction in a case in which an operator uses the radiation-irradiation device 1 as shown in Fig. 2 and in a case in which an operator transports the radiation-irradiation device 1 while viewing the monitor 23 as described later, the "right side" of the radiation-irradiation device 1 indicates the side corresponding to -x direction of Fig. 1, and the "left side" of the radiation-irradiation device 1 indicates the side corresponding to +x direction of Fig. 1.

As shown in Figs. 1 and 2, the radiation-irradiation device 1 of this embodiment includes a leg unit 10, a body unit 20, an arm unit 30 serving as a radiation source holding unit, and a radiation source unit (radiation source) 40.

The leg unit 10 can travel on a device-placement surface 2, and includes a plate-like base 11 and four wheel parts 12 that are mounted on four corners of the lower surface of the base 11. As in the case of a general caster, each of the wheel parts 12 is composed of a wheel, such as a rubber tire, a revolving portion that holds the wheel so as to allow the wheel to be rotatable, and the like. The revolving portion is mounted on the base 11 so as to be revolvable about an axis, which extends in the vertical direction, in a horizontal plane. Accordingly, the leg unit 10 is adapted to be capable of traveling on the device-placement surface 2 in an arbitrary direction.

The body unit 20 is installed on the leg unit 10, and includes a housing 21. A control unit 22, which controls the drive of the radiation-irradiation device 1, and a battery (not shown) are received in the housing 21. The control unit 22 is a unit that is used to control not only the dose, the quality, the irradiation time, and the like of radiation generated from the radiation source unit 40 but also various operations of the radiation-irradiation device 1. The control unit 22 is composed of, for example, a computer in which a program for control is installed, dedicated hardware, or a combination of both the computer and the dedicated hardware. Further, a monitor 23 in which a speaker 18 is built is mounted on the upper surface of the housing 21. A handle 26, which is used to push or pull the radiation-irradiation device 1, is mounted on the upper portion of the housing 21 through an adapter 27. Furthermore, cameras 28A, 28B, 28C, and 28D, which are used to take an omnidirectional image of the device 1, are mounted on the right side surface, the rear surface, the left side surface, and the front surface of the body unit 20, respectively. For example, a digital video camera, which outputs digital signals representing a taken video, is used as each of these cameras 28A to 28D.

The monitor 23 serving as display means is formed of a liquid crystal panel or the like, and displays a radiation image that is acquired from the imaging of a subject H and various kinds of information that is required for the control of the device 1. Further, the monitor 23 includes a touch panel type input unit 24, and receives the input of various commands required for the operation of the device 1. The control unit 22 functions as a console (operator console), which is used to command various operations of the radiation-irradiation device 1, together with the input unit 24. The control unit 22 is formed separately from the input unit 24 in this embodiment, but the control unit 22 and the input unit 24 may be integrated with each other. The monitor 23 is mounted on the upper surface of the body unit 20 so that the inclination and the rotational position of the monitor 23 are changeable. Further, the monitor 23 may be detachably mounted on the body unit 20. In a case in which the monitor 23 is separate from the body unit 20, the monitor 23 and the control unit 22 are adapted to communicate with each other by radio.

The arm unit 30 is held on the body unit 20. In detail, the arm unit 30 is held on the surface of the body unit 20 opposite to the handle 26, that is, a right surface of the body unit 20 in Fig. 2. The arm unit 30 is adapted to be capable of being raised and lowered relative to the body unit 20 by a raising/lowering mechanism 50 that is formed of, for example, a pantograph mechanism or the like. The arm unit 30 includes a first arm 31, a second arm 32 a first rotational moving portion 33, a second rotational moving portion 34, and a radiation source holding part 35. The radiation source unit 40 is held at the distal end of the first arm 31 through the radiation source holding part 35. In the following description, an end portion of the first arm 31 close to the radiation source unit 40 is referred to as an upper end portion and an end portion of the first arm 31 close to the second arm 32 is referred to as a lower end portion. Further, an end portion of the second arm 32 close to the first arm 31 is referred to as an upper end portion and an end portion of the second arm 32 close to the body unit 20 is referred to as a lower end portion.

The first and second arms 31 and 32 are connected to each other by the first rotational moving portion 33 so as to be rotationally movable about a rotational movement axis AX1. The rotational movement axis AX1 is an axis extending in the x direction. The first arm 31 is rotationally moved about the rotational movement axis AX1 so that an angle between the first and second arms 31 and 32 is changed. The first rotational moving portion 33 holds both the first and second arms 31 and 32 so that the first arm 31 is rotationally moved relative to the second arm 32 through a friction mechanism. For this reason, the first arm 31 is rotationally movable in a case in which an external force, which is strong to some extent, is applied to the first arm 31, and maintains an angle relative to the second arm 32 without being rotationally moved as long as an external force is not applied to the first arm 31.

The second arm 32 is connected to an adapter 51, which is mounted on the upper end portion of the raising/lowering mechanism 50, through the second rotational moving portion 34 so as to be rotationally movable about a rotational movement axis AX2. The rotational movement axis AX2 is an axis extending in the x direction. The second arm 32 is rotationally moved in a YZ plane about the rotational movement axis AX2 so that an angle between the second arm 32 and the surface of the body unit 20 on which the arm unit 30 is held is changed. The second rotational moving portion 34 holds both the second arm 32 and the adapter 51 so that the second arm 32 is rotationally moved relative to the adapter 51 through a friction mechanism. For this reason, the second rotational moving portion 34 is rotationally movable in a case in which an external force, which is strong to some extent, is applied to the second rotational moving portion 34, and maintains an angle relative to the body unit 20 without being rotationally moved as long as an external force is not applied to the second rotational moving portion 34.

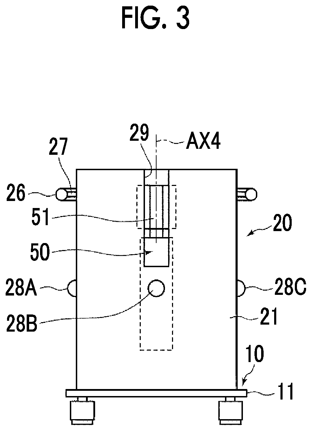

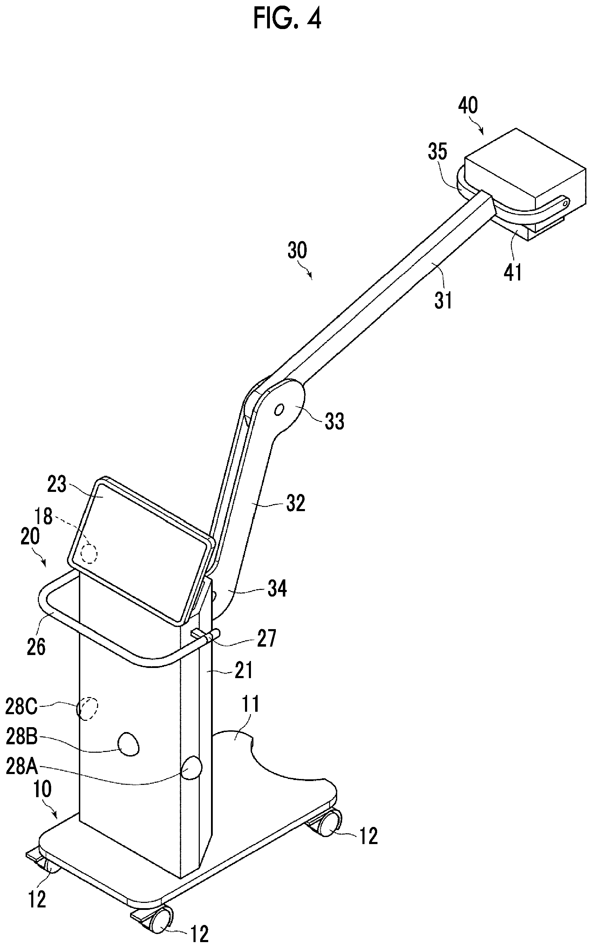

Fig. 3 is a diagram of a part of the radiation-irradiation device 1 viewed in the direction of an arrow A of Fig. 2. As shown in Fig. 3, a groove 29, through which the adapter 51 can pass at the time of an operation for raising and lowering the arm unit 30 performed by the raising/lowering mechanism 50, is formed on the right surface of the body unit 20 in Fig. 2. For illustration, the monitor 23 and the arm unit 30 are not shown in Fig. 3. Further, in a case in which the radiation-irradiation device 1 is used, the arm unit 30 is shifted from the initial position to a use position at which the first and second arms 31 and 32 are unfolded. Fig. 4 shows the radiation-irradiation device 1 of which the arm unit 30 is set to one example of the use position. Description will also be continued below with reference to Fig. 4.

The radiation source holding part 35 is formed in a substantially U shape, and is mounted on the distal end of the first arm 31. The radiation source unit 40 is connected to the distal end of the first arm 31 through the radiation source holding part 35 so as to be rotationally movable about a rotational movement axis AX3. The rotational movement axis AX3 is an axis extending in the x direction. The radiation source unit 40 is rotationally moved about the rotational movement axis AX3 so that an angle between the radiation source unit 40 and the first arm 31 is changed. The radiation source holding part 35 holds both the radiation source unit 40 and the first arm 31 so that the radiation source unit 40 is rotationally moved relative to the first arm 31 through a friction mechanism. For this reason, the radiation source unit 40 is rotationally movable in a case in which an external force, which is strong to some extent, is applied to the radiation source unit 40, and maintains an angle relative to the first arm 31 without being rotationally moved as long as an external force is not applied to the radiation source unit 40.

Furthermore, the adapter 51 is connected to the raising/lowering mechanism 50 so as to be rotationally movable about a rotational movement axis AX4. The rotational movement axis AX4 is an axis extending in the z direction. The adapter 51 is rotationally moved in an xy plane about the rotational movement axis AX4. In a case in which the adapter 51 is rotationally moved in this way, the entire arm unit 30 is rotationally moved in the xy plane. The adapter 51 holds both the second arm 32 and the raising/lowering mechanism 50 so that the second arm 32 is rotationally moved relative to the raising/lowering mechanism 50 through a friction mechanism. For this reason, the arm unit 30 connected to the adapter 51 is rotationally movable in a case in which an external force, which is strong to some extent, is applied to the arm unit 30, and maintains a rotational movement position thereof in the xy plane without being rotationally moved as long as an external force is not applied to the arm unit 30.

As described above, in this embodiment, each of the rotational movement of the first arm 31 relative to the second arm 32, the rotational movement of the second arm 32 relative to the body unit 20 in the yz plane, the rotational movement of the radiation source unit 40 relative to the first arm 31, and the rotational movement of the entire arm unit 30 relative to the raising/lowering mechanism 50 in the xy plane is achieved through the friction mechanism. However, rotational movement positions may be fixed by publicly known lock mechanisms. In this case, it is possible to rotationally move the first arm 31, the second arm 32, the radiation source unit 40, and the entire arm unit 30 by releasing the lock mechanisms. Further, it is possible to fix the rotational movement positions by locking the lock mechanisms at desired rotational movement positions.

Here, in a case in which the radiation-irradiation device 1 shown in Fig. 1 is not in use, the arm unit 30 is positioned at an initial position. The initial position of the arm unit 30 is a position where the entire arm unit 30 is present so as to be away from the raising/lowering mechanism 50 in the y direction and is positioned at the lowest position among the positions where the arm unit 30 is moved in the vertical direction by the raising/lowering mechanism 50 in a state in which the first and second arms 31 and 32 are folded. Particularly, in this embodiment, the initial position is set to the position of the arm unit 30 in a state in which the first and second arms 31 and 32 are folded to a limit where the first and second arms 31 and 32 are not rotationally moved any more as shown in Fig. 1. At the initial position, the second arm 32 is in a state in which the first rotational moving portion 33 is positioned above the second rotational moving portion 34.

Here, a position where the entire arm unit 30 is present so as to be away from the raising/lowering mechanism 50 in the +y direction is referred to as the initial rotational movement position of the arm unit 30. The +y direction is a right direction in Fig. 2. The arm unit 30 is rotationally movable about the rotational movement axis AX4 from the initial rotational movement position in the xy plane as described above. The rotational movement is performed about the rotational movement axis AX4 in the range of 45° in a clockwise direction to 45° in a counterclockwise direction.

The first and second arms 31 and 32 can be fastened to each other by a fastening belt 36 at the initial position. For example, one end portion of the fastening belt 36 is mounted on the second arm 32 and a hook-and-loop fastener is mounted on the other end portion of the fastening belt 36. A hook-and-loop fastener corresponding to the hook-and-loop fastener of the fastening belt 36 is mounted on the surface of the first arm 31 opposite to the surface of the first arm 31 shown in Fig. 1. Further, the fastening belt 36 is put around the first arm 31 from the right surface of the first arm 31 in Fig. 1 to the opposite surface of the first arm 31 to connect the hook-and-loop fastener of the fastening belt 36 to the hook-and-loop fastener mounted on the first arm 31. Accordingly, the first arm 31 is not rotationally moved relative to the second arm 32 at the initial position.

The radiation source unit 40 has a structure where a radiation source, a collimator for narrowing the irradiation range of radiation, and the like are received in a housing 41. The radiation source is composed of, for example, an X-ray tube, a booster circuit, cooling means for cooling the X-ray tube, and the like. The emission of radiation from the radiation source of the radiation source unit 40 is performed by a command that is input from the input unit 24 of the monitor 23 by an operator. The input unit 24 is used to input information that is required to perform various operations of the radiation-irradiation device 1, and forms a console (operator console), which is used to perform the management of imaging orders, the image processing of a taken image, the display of the taken image, and the like, together with the control unit 22 and the monitor 23.

In this embodiment, in a case in which the radiation image of a subject H is to be taken, a radiation detector 80 is disposed under a subject H supine on a bed 3 as shown in Fig. 2 and the radiation detector 80 is irradiated with radiation, such as, X-rays, emitted from the radiation source of the radiation source unit 40 and passing through the subject H.

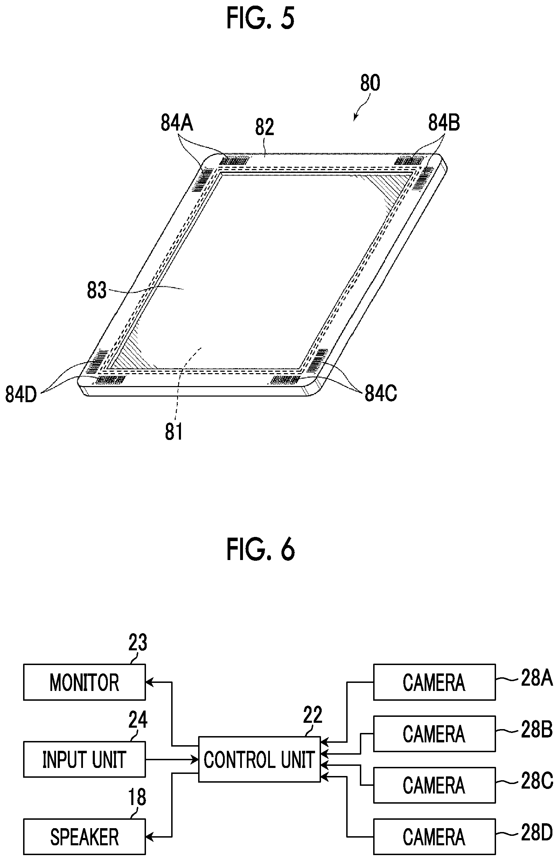

The radiation detector 80 will be briefly described here with reference to Fig. 5. Fig. 5 is a perspective view showing the appearance of the radiation detector viewed from a front surface that is a radiation-irradiation side. As shown in Fig. 5, the radiation detector 80 of this embodiment is a cassette-type radiation detector that includes an image detection unit 81 serving as a radiation image recording medium and a housing 82 receiving the image detection unit 81. As is well known, the image detection unit 81 includes a scintillator (phosphor) that converts incident radiation into visible light and a thin-film-transistor (TFT) active matrix substrate. A rectangular imaging region in which a plurality of pixels for accumulating electric charges corresponding to visible light emitted from the scintillator are arranged is formed on the TFT active matrix substrate. In the radiation detector 80, the image detection unit 81 is a radiation image recording medium. However, for convenience' sake, the entire radiation detector 80 may also be called as a radiation image recording medium in this specification.

An imaging control unit, which includes a gate driver, a signal processing circuit, and the like, and the like are built in the housing 82 in addition to the image detection unit 81. The gate driver applies gate drive signals to a gate of a TFT to switch the TFT. The signal processing circuit converts electric charges, which are accumulated in the pixels, into analog voltage signals by an amplifier, obtains digital image signals representing an X-ray image through the AD conversion of the analog voltage signals, and stores the digital image signals serving as radiation image information in a memory. The digital image signals may be stored in a memory that is built in the radiation detector 80, or may be transmitted to an external console by wire communication or radio communication. Further, the housing 82 has substantially the same size as, for example, a film cassette using a silver salt photograph film as a recording medium or a computed radiography (CR) cassette using an imaging plate (IP) as a recording medium that is based on International Organization for Standardization (ISO) 4090:2001.

Markers 84A to 84D, which represent identification information for identifying the radiation detector 80, are given to four corners of the front surface of the housing 82. In this embodiment, each of the markers 84A to 84D is formed of two bar codes orthogonal to each other. The above-mentioned four markers do not necessarily need to be provided, and, for example, one marker, two markers, or the like may be provided. Further, the installation positions of the markers are not limited to the corners of the housing 82, and the markers may be installed at side portions of the housing 82. Furthermore, the markers 84A to 84D may be adapted to transmit identification information by radio.

Next, an operation in a case in which a radiation image is not yet taken by the radiation-irradiation device 1 of this embodiment will be described. In the state which is shown in Fig. 1 and in which the radiation-irradiation device 1 is not in use, that is, in a state in which the arm unit 30 is stored, the radiation-irradiation device 1 is transported to a use position while being made to travel on the device-placement surface 2, such as the floor of a hospital, by the wheel parts 12 of the leg unit 10. The transport of the radiation-irradiation device 1 is performed by a transport force for pushing or pulling the radiation-irradiation device 1 that is applied by an operator (device user) while the operator holds the handle 26.

Here, "a state in which the arm unit 30 is stored" is a state in which the arm unit 30 is positioned at the above-mentioned initial position. In a case corresponding to emergency care or the like, the radiation-irradiation device 1 may be carried to the use position in the same manner as described above in a state in which the arm unit 30 is unfold. Since each of the wheel parts 12 is revolvably mounted on the base 11 as described above, the radiation-irradiation device 1 can be moved in a front-back direction and the lateral direction and can also be moved along a large curve. In addition, the radiation-irradiation device 1 can also revolve at that position. Accordingly, the radiation-irradiation device 1 can be quickly transported to a use position in a state in which the radiation-irradiation device 1 revolves in a small radius.

The taking of a radiation image is performed on the subject H who is supine on, for example, the bed 3 as shown in the above-mentioned Fig. 2. In a case in which the radiation-irradiation device 1 is to be disposed close to the subject H, the radiation-irradiation device 1 can also be moved in the height direction of the subject H by the wheel parts 12. Accordingly, the radiation-irradiation device 1 can be easily disposed at the optimum position.

Next, a transport assisting method for a radiation-irradiation device according to an embodiment of the invention will be described. The transport assisting method is to perform notification, that is, to display a movement path, which allows the radiation-irradiation device 1 not to collide with an obstacle, on the monitor 23 or to notify an operator of the movement path with a voice by a speaker 18 in a case in which the radiation-irradiation device 1 is to be transported to a position (target position) where irradiation using radiation is performed by a transport force of the operator as described above.

Fig. 6 is a block diagram showing the basic configuration of a transport assisting device that performs notification. The transport assisting device includes: the cameras 28A, 28B, 28C, and 28D that are mounted on the right side surface, the rear surface, the left side surface, and the front surface of the body unit 20 as described above, respectively; the above-mentioned control unit 22 that receives the outputs of these cameras 28A to 28D; the above-mentioned monitor 23 that receives image signals output by the control unit 22; the above-mentioned speaker 18 that receives voice signals output by the control unit 22; and the above-mentioned input unit 24 that is used to input various commands to the control unit 22.

In this embodiment, target position setting means, current position detecting means, and obstacle position detecting means of the transport assisting device of the invention are formed by the cameras 28A to 28D and the control unit 22. A plurality of cameras, which take stereo images corresponding to the four directions of the radiation-irradiation device 1, and the like can also be used as the obstacle position detecting means. The plurality of cameras may be mounted on the body unit 20 and may also be mounted on the radiation source unit 40. Further, the cameras 28A to 28D, the control unit 22, the monitor 23, and the speaker 18 form notification means of the transport assisting device of the invention.

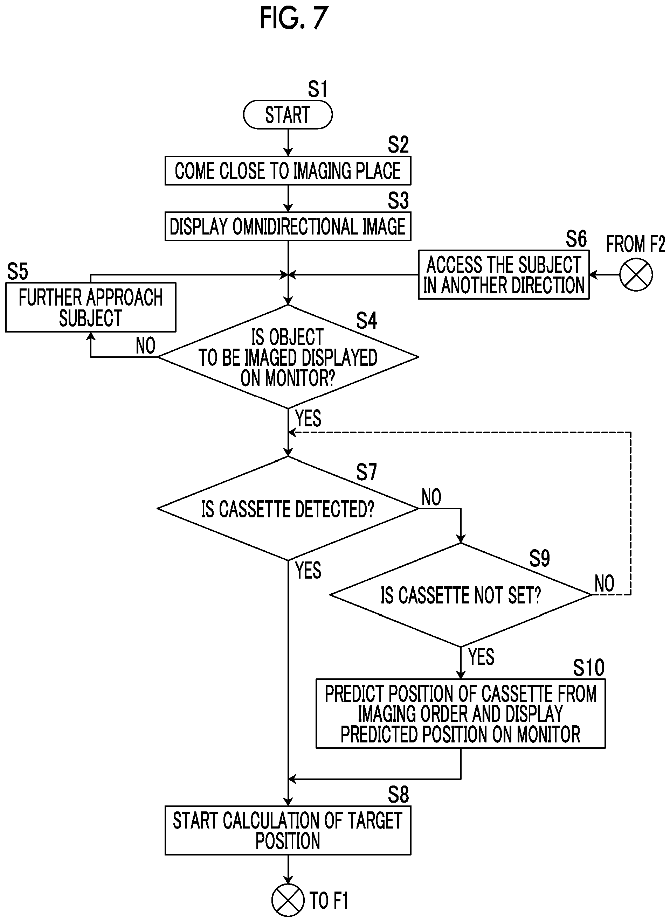

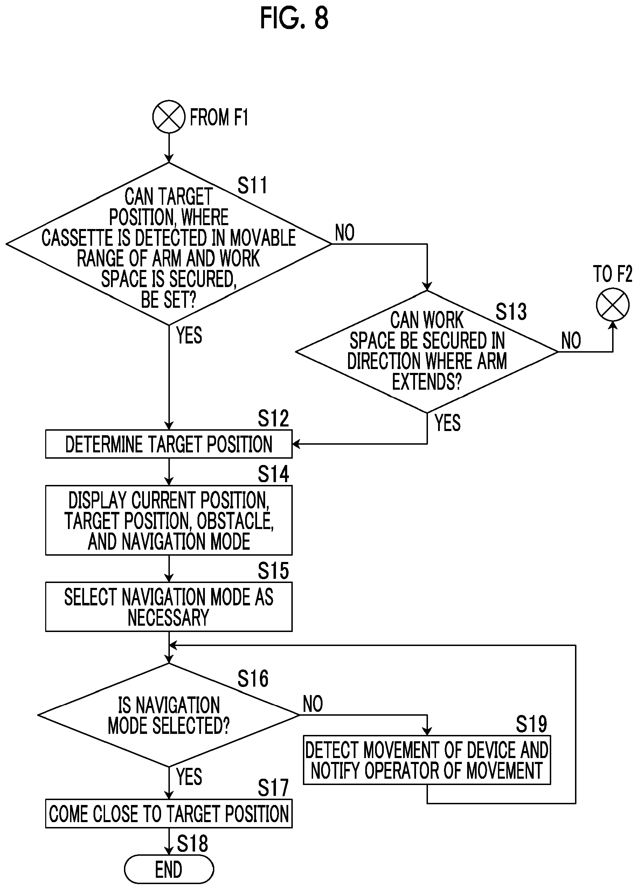

Next, the transport of the radiation-irradiation device 1 in a case in which the transport assisting method is performed will be described with reference to Figs. 7 and 8. In this embodiment, the transport assistance of the radiation-irradiation device 1 is selectively performed in either an "obstacle approach mode" in which the transport assistance of the radiation-irradiation device 1 is started in a case in which the radiation-irradiation device 1 comes close to an obstacle or a "target position approach mode" in which the transport assistance of the radiation-irradiation device 1 is started in a case in which the radiation-irradiation device 1 comes close to a target position to be described later. Figs. 7 and 8 show the flow of processing until the two modes are selected and the transport assistance ends.

As shown in Fig. 7, the transport of the radiation-irradiation device 1 is started first in Step S1 after the power source of the radiation-irradiation device 1 is turned on, and the radiation-irradiation device 1 comes close to an imaging place, at which a radiation image is to be taken, by a transport force of an operator (Step S2). The imaging place is, for example, a place close to a bed in an emergency room and a reader for an integrated circuit (IC) tag is installed close to the bed. The radiation-irradiation device 1 includes an IC tag in which identification information about the radiation-irradiation device 1 and other information are recorded, and the reader is to read the information recorded in the IC tag. An example of the arrangement of the emergency room, the bed, and the reader will be described in detail later.

In a case in which the radiation-irradiation device 1 comes close to the imaging place in Step S2, the information recorded in the IC tag of the radiation-irradiation device 1 is read by the reader. Which mode of the two modes takes priority is registered in the radiation-irradiation device 1 and the reader as a default condition. Generally, the "obstacle approach mode" is set as a prescribed condition in a radiation-irradiation device 1 of which the field of view on the front side is not good, and the "obstacle approach mode" is set as a default mode in a case in which, for example, an emergency room or the like serving as an imaging site is in disorder even in a radiation-irradiation device 1 of which the field of view on the front side is good. Generally, the setting of the two modes is not registered in the reader, and the default mode of the radiation-irradiation device 1 is basically employed in the reader. However, in a case in which a mode is set in the reader, the default mode of the radiation-irradiation device 1 is ignored and the mode set in the reader is employed.

In a case in which the power source of the radiation-irradiation device 1 is turned on, an image showing the entire circumference of the radiation-irradiation device 1, that is, an omnidirectional image is displayed on the monitor 23 (Step S3). In a case in which the transport assisting method of the invention is to be performed, an operator transports the radiation-irradiation device 1 while viewing the image displayed on the monitor 23. Accordingly, the transport direction of the radiation-irradiation device 1 in this case is the +y direction in Fig. 1. That is, the +y direction corresponds to the front side in the transport direction.

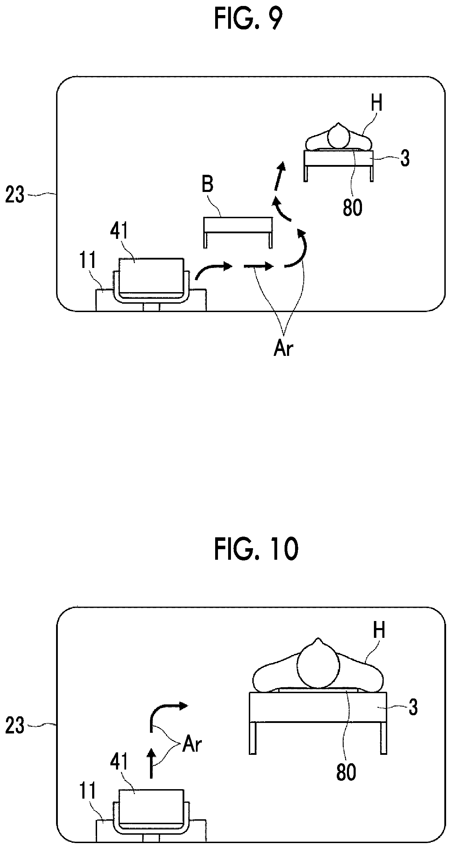

Here, the omnidirectional image will be described. The control unit 22 shown in Fig. 6 reconstructs the omnidirectional image of the radiation-irradiation device 1 from a body-right-side image, a body-rear-side image, a body-left-side image, and a body-front-side image that are taken by the cameras 28A to 28D, respectively. A part of the radiation-irradiation device 1 is also displayed in the reconstructed omnidirectional image. The omnidirectional image is displayed on the monitor 23. Examples of the image displayed on the monitor 23 are shown in Figs. 9 and 10. The images shown in Figs. 9 and 10 correspond to an image showing the circumstances on the front side of an automobile in an example of a car navigation system, and are displayed using only a part of an omnidirectional image (an image showing only the front side in this way is also referred to as an omnidirectional image for convenience' sake). In addition, in this embodiment, an omnidirectional image corresponding to an image obtained by viewing an automobile, which is being driven, from above can also be displayed on the monitor 23 in the example of a car navigation system likewise. This omnidirectional image will be described in detail later.

In a case in which an omnidirectional image is to be displayed on the monitor 23, the control unit 22 detects the markers 84A to 84D, which are to be given to the housing 82 of the radiation detector 80 as described above, by, for example, image processing and makes the monitor 23 display an omnidirectional image corresponding to a direction in which these markers 84A to 84D are included, that is, a direction in which the radiation detector 80 is included. Accordingly, if the radiation-irradiation device 1 reaches a position where the radiation-irradiation device 1 can detect the radiation detector 80, the subject H and the bed 3 present on and under the radiation detector 80 are also displayed on the monitor 23 together with the radiation detector 80 as shown in Figs. 9 and 10. The positions of the radiation detector 80, the subject H, and the bed 3 are used to calculate a position where the radiation-irradiation device 1 should finally reaches, that is, a target position. Since the omnidirectional image shown in Fig. 10 is an omnidirectional image that is obtained at a point of time when the radiation-irradiation device 1 further approaches the radiation detector 80, the subject H, and the bed 3 in comparison with the omnidirectional image shown in Fig. 9, the radiation detector 80, the subject H, and the bed 3 are displayed to be larger in the omnidirectional image shown in Fig. 10.

It is preferable that a motion sensor is built in the housing 82. In this case, the position of the radiation detector 80 can be traced on the basis of the initial positions of the markers 84A to 84D and motion signals output from the motion sensor in a case in which the markers 84A to 84D are hidden from the subject H and cannot be detected. In addition, means for transmitting the individual information of the radiation detector 80 by radio may be provided in the housing 82, and the position of the radiation detector 80 may be detected from the radio signal of the means.

In this case, the base 11 of the radiation-irradiation device 1 and the housing 41 of the radiation source unit are further also displayed on the monitor 23. The display of the base 11 and the housing 41 corresponds to the display of the current position of the radiation-irradiation device 1. Further, if an obstacle B, such as a bed, is present, the obstacle B is also displayed on the monitor 23. The display of the obstacle B corresponds to the display of the position of the obstacle. Furthermore, after the transport assistance to be described later is started, for example, a path shown by arrows Ar and the like, which indicates an appropriate movement path for the transport of the radiation-irradiation device 1, that is, a movement path that allows the radiation-irradiation device 1 not to collide or interfere with the obstacle B, is displayed on the monitor 23. This movement path is a movement path that is set even in consideration of the traveling performance of the radiation-irradiation device 1, and does not necessarily means a straight line path from the current position of the radiation-irradiation device 1 to a target position.

Returning to Fig. 7, in Step S4, an operator determines whether or not the subject H and the like, which are objects to be imaged, are displayed on the monitor 23. Generally, this determination corresponds to YES, that is, the subject H and the like are displayed. However, if the subject H and the like are not displayed, the operator further transports the radiation-irradiation device 1 in a direction in which the radiation-irradiation device 1 approaches the subject H (Step S5). The operator makes the radiation-irradiation device 1 approach the subject H in another direction in Step S6 before the determination of Step S4, but this will be described later. If the subject H and the like are displayed on the monitor 23, here, the radiation detector 80 is generally set on the bed 3 and under the subject H.

In the next step S7, the control unit 22 determines whether or not the markers 84A to 84D, that is, the radiation detector 80 is detected in the above-mentioned manner. In Figs. 7 and 8, the radiation detector 80 is written and shown as a "cassette". If the radiation detector 80 is detected in Step S7, the control unit 22 then starts the calculation of a target position in Step S8. The calculation of the target position is basically performed on the basis of the position of the radiation detector 80 in the above-mentioned reconstructed image.

If the radiation detector 80 is not detected in Step S7, the control unit 22 makes the monitor 23 display "Don't you set the cassette?" and makes the monitor 23 display "YES" and "NO" in Step S9. The operator views the display of these and presses the display of "NO" to input the fact of "NO" to the control unit 22 from the input unit 24 if the operator forgets to set the radiation detector 80. Further, the operator performs the setting of the radiation detector 80 that has been forgotten. In a case in which the radiation detector 80 is set in this way, the radiation detector 80 is detected in Step S7.

In a case in which the operator is to postpone the setting of the radiation detector 80 due to certain circumstances, the operator presses the display of "YES" to input the fact of "YES" to the control unit 22 from the input unit 24. In this case, in Step S10, the control unit 22 predicts the position of the radiation detector 80 on the basis of the position of the detected subject H from an imaging order for the subject H and makes the monitor 23 display the predicted position. That is, since, for example, information about portions to be imaged, such as a "frontal chest in standing position" and "frontal abdomen in supine position", an imaging posture, and an imaging direction is generally included in the imaging order, it is possible to predict the position of the radiation detector 80 to be set relative to the position of the subject H in a case in which the information is used. Further, in a case in which the predicted position of the radiation detector 80 is to be displayed on the monitor 23, the fact that the position of the radiation detector 80 is a predicted position and the setting of the radiation detector 80 is facilitated is displayed on the monitor 23 together with the predicted position. If the radiation detector 80 is not in the movable range of the arm unit 30 to be described later in a case in which the setting of the radiation detector 80 is performed, it is preferable that warning sound is generated from the speaker 18 or a warning is displayed on the monitor 23. In a case in which a display is made on the monitor 23, the control unit 22 then starts the calculation of a target position in Step S8 like in a case in which the radiation detector 80 is detected in Step S7.

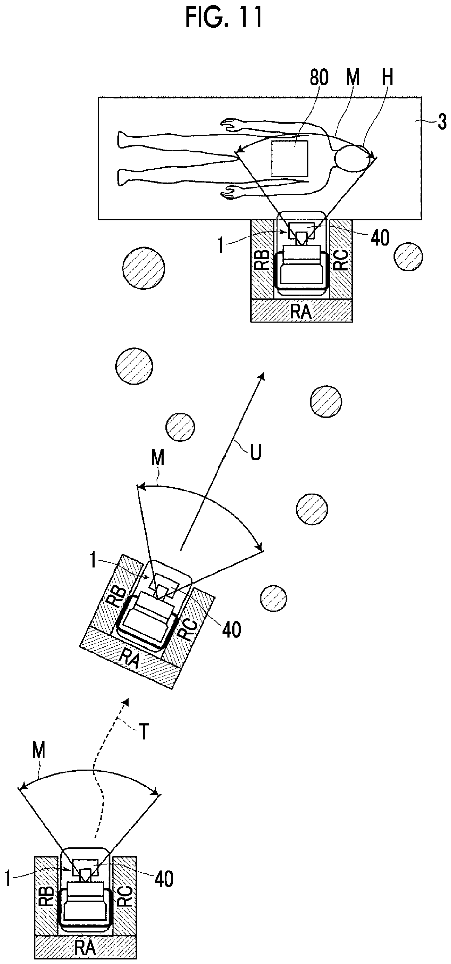

In a case in which the control unit 22 starts the processing of Step S8, the control unit 22 determines whether or not a target position where the radiation detector 80 can be detected in the movable range of the arm unit 30 and work spaces can be secured around the radiation-irradiation device 1 can be set in Step S11 shown in Fig. 8. Here, in detail, "detects the radiation detector 80 in the movable range of the arm unit 30" means that the radiation source unit 40 can be disposed at a radiation-irradiation position where a radiation image with respect to the radiation detector 80 can be taken in the movable range where the position of the radiation source unit 40 can be changed by the arm unit 30. The movable range of the arm unit 30 of this embodiment is the rotational movement range of the arm unit 30 that is shown in Fig. 11 by an arrow M, and this movable range is the rotational movement range of the arm unit 30 about the rotational movement axis AX4 shown in Fig. 2.

Further, three regions RA, RB, and RC shown in Fig. 11, which are determined in advance, are assumed as the "predetermined work spaces" in this embodiment. The region RA is a work space that is set on the rear side of the radiation-irradiation device 1 in the transport direction, the region RB is a work space that is set on the left side of the radiation-irradiation device 1, and the region RC is a work space that is set on the right side of the radiation-irradiation device 1. Among these work spaces, the work space of the region RA is a work space that is essential for not only work for transporting the radiation-irradiation device 1 but also work relating to the taking of a radiation image. On the other hand, the work space of the region RB and the work space of the region RC are spaces that are required to perform work relating to the taking of a radiation image from the sides of the radiation-irradiation device 1. It is particularly preferable that both the work space of the region RB and the work space of the region RC are secured, but it is generally preferable that at least only one thereof is secured. Specifically, each of the regions RA, RB, and RC is set to a size that allows at least one person to easily pass. Further, it is more preferable that the size to which the region is to set can be appropriately changed according to the body type of the operator.

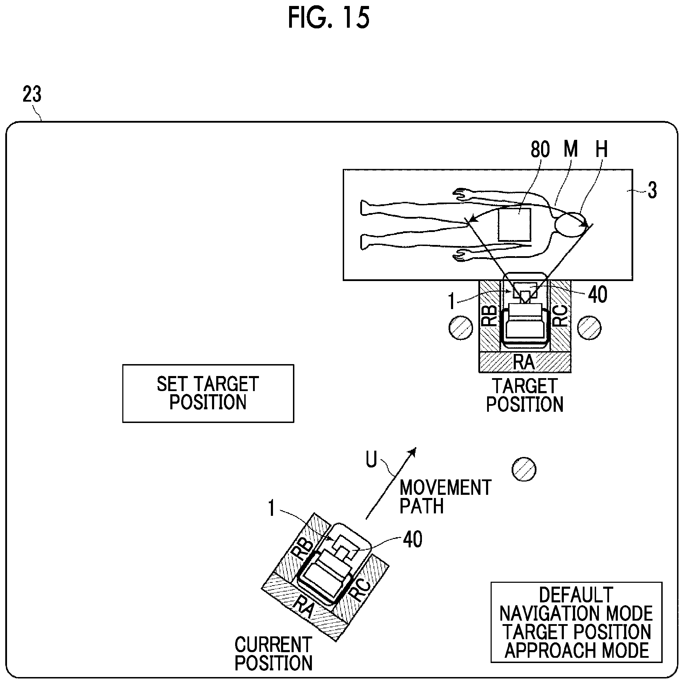

If the determination of Step S11 corresponds to YES, the control unit 22 calculates and sets a target position and determines the target position as a target position in Step S12. A detailed timing where the target position is set will be described in detail later. Further, in a case in which the target position is determined, an overhead omnidirectional image shown in, for example, Fig. 15 is displayed on the monitor 23. As shown here, the radiation-irradiation device 1, the regions RA, RB, and RC as the work spaces that are set on the three sides of the radiation-irradiation device 1, and the rotational movement range of the arm unit 30 that is shown by an arc-shaped arrow M and segments positioned on both sides of the arrow M are displayed in the overhead omnidirectional image together with the subject H, the radiation detector 80, and the bed 3. Furthermore, in a case in which obstacles (shown in Fig. 15 as hatched circles) are present, the obstacles are also displayed.

An appropriate movement path shown in Fig. 15 by a solid line U and the display of "movement path" showing that the solid line U indicates the appropriate movement path are shown in an omnidirectional image, which is obtained in a case in which a target position is set, together with the display of "target position was set", "target position", "current position", and "default navigation mode: target position approach mode".

If the determination of Step S11 corresponds to NO, the control unit 22 in Step S13 determines whether or not work spaces can be secured in a direction where the first and second arms 31 and 32 extend. The reason for this is that the radiation detector 80 can be detected in the movable range of the arm unit 30 and predetermined work spaces can be secured around the radiation-irradiation device 1 if the first and second arms 31 and 32 extend even in a case in which the determination of Step S11 corresponds to NO in a state in which the first and second arms 31 and 32 are stored.

If the determination of Step S13 corresponds to YES, the control unit 22 determines a target position in Step S12. If the determination of Step S13 corresponds to NO, the operator makes the radiation-irradiation device 1 come close to a target position in a direction separate from a direction scheduled at the beginning (Step S6 shown in Fig. 7). Accordingly, the flow of processing returns to Step S4 shown in Fig. 7.

In a case in which a target position is determined in Step S12 of Fig. 8, in Step S14, the control unit 22 makes the monitor 23 display the current position of the radiation-irradiation device 1, the determined target position, and obstacles and makes the monitor 23 display the above-mentioned default mode, that is, one of the "obstacle approach mode" and the "target position approach mode". In a case in which, for example, the default mode is the "target position approach mode", the display of the monitor 23 at this time corresponds to "RADIATION-IRRADIATION DEVICE: TARGET POSITION APPROACH MODE, No.5 BED: No MODE SETTING; A TARGET POSITION APPROACH MODE WAS EMPLOYED ACCORDINGLY".

In Fig. 8, the two modes are collectively written and shown as a "navigation mode". The operator views the display of these, selects the other mode, which is not the default mode, (in this case, the "obstacle approach mode") in Step S15 as necessary, and inputs the selected mode to the control unit 22 from the input unit 24. For example, if many obstacles are present in a case in which the operator views the display of the monitor 23 at the time of setting of a target position shown in Fig. 15, the operator selects the "obstacle approach mode" in Step S15. The selection and input of the mode are performed through the selection of one of the two mode-name buttons displayed on, for example, the touch panel type monitor 23 and the touch of the selected mode-name button.

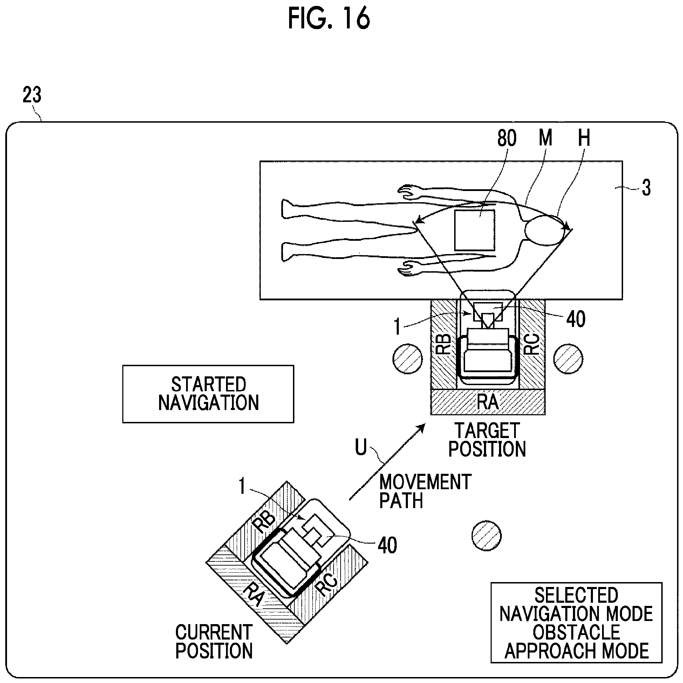

Next, in Step S16, the control unit 22 determines whether or not the other mode is selected. If the control unit 22 determines that the other mode is selected, the control unit 22 makes the monitor 23 display, for example, "RADIATION-IRRADIATION DEVICE: TARGET POSITION APPROACH MODE, No.8 BED: OBSTACLE APPROACH MODE, AN OBSTACLE APPROACH MODE WAS EMPLOYED ACCORDINGLY". While the operator views the screen of the monitor 23 and confirms that a mode is the obstacle approach mode, the operator transports the radiation-irradiation device 1 so that the radiation-irradiation device 1 comes closer to a target position (Step S17). In a case in which the transport of the radiation-irradiation device 1 is continued, the radiation-irradiation device 1 approaches an obstacle. In this state, the control unit 22 makes the monitor 23 display an omnidirectional image shown in Fig. 16. In this case, an appropriate movement path shown in Fig. 16 by a solid line U and the display of "movement path" showing that the solid line U indicates the appropriate movement path are shown on the monitor 23 together with the display of "navigation is started", "target position", "current position", and "selected navigation mode: obstacle approach mode". Further, the control unit 22 performs the guidance of transport using a voice of "go forward please" together with the display of the movement path on the monitor 23. In case in which the control unit 22 performs guidance using the display of an image and guidance using a voice in parallel as described above, the operator can grasp an appropriate movement path even though the operator misses the guidance for the appropriate movement path without viewing the display or listening the voice. Particularly, since the attention of the operator tends to be caught by the viewing of an obstacle in a case in which the radiation-irradiation device 1 is close to the obstacle, it is preferable that guidance using the notification of the voice of the speaker 18 is performed. The same applies to a case in which any of the target position approach mode and the obstacle approach mode is set.

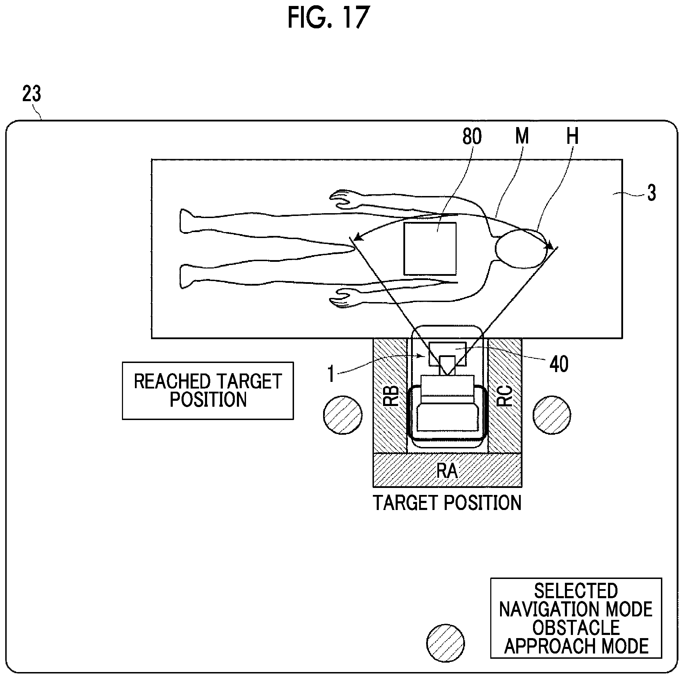

The transport of the radiation-irradiation device 1 ends in a case in which the radiation-irradiation device 1 reaches a target position (Step S18). Fig. 17 shows an example of the display screen of the monitor 23 in a case in which the transport of the radiation-irradiation device 1 ends.

An operation for selecting the other mode instead of the default mode may not be performed in Step S15 as long as the operation for selecting the other mode instead of the default mode is not particularly needed. In this case, it is naturally determined in Step S16 that the other mode is not selected. In a case in which it is determined that the other mode is not selected, the control unit 22 makes the monitor 23 display notification, such as "the obstacle approach mode is not selected", or notifies the operator of the fact of "the obstacle approach mode is not selected" with a voice by a speaker 18 after the control unit 22 detects the movement of the radiation-irradiation device 1 in Step S19. After a worker confirms the notification of the fact, the worker continues to transport the radiation-irradiation device 1 so that the radiation-irradiation device 1 comes closer to a target position. That is, in a case in which the other mode is not selected, that is, in a case in which a prescribed mode is set, the processing of Steps S16 and the processing of Step S19 are repeated but the operator can continue to transport the radiation-irradiation device 1 regardless of the repetition of the processing.

The transport assistance of the radiation-irradiation device 1 to be performed in the "obstacle approach mode" or the "target position approach mode" will be described in detail with reference to Figs. 11 to 14. All of the display of the monitor 23 and the output of a voice of the speaker 18, which relate to the transport assistance to be described below, are basically performed on the basis of the commands of the control unit 22.

The "obstacle approach mode" is a mode in which the transport assistance of the radiation-irradiation device 1 is started in a case in which the radiation-irradiation device 1 comes close to an obstacle and is a mode suitable for a case in which the forward visibility of the radiation-irradiation device 1 is poor. Fig. 11 shows an example of a change in the transport position of the radiation-irradiation device 1 in this mode. Obstacles are shown in Fig. 11 as hatched circles. Fig. 11 shows a state in which the position of the radiation-irradiation device 1 is viewed from above. However, as described above, an omnidirectional image shown in this state is also displayed on the monitor 23 instead of the omnidirectional images shown in the states of Figs. 9 and 10. In this case, two omnidirectional images are switched and displayed in a case in which, for example, the operator inputs a command for switching the display state of an omnidirectional image from the input unit 24.

For illustration, three positions of the radiation-irradiation device 1, which are changed with time, are shown in Fig. 11. However, only the radiation-irradiation device 1, which is present at the current position not only in a case in which an omnidirectional image is displayed but also at a point of time of the display of the omnidirectional image, is displayed in real time. Fig. 15 shows an example of the omnidirectional image that is displayed on the monitor as described above. As shown here, the radiation-irradiation device 1, the regions RA, RB, and RC as the work spaces that are set on the three sides of the radiation-irradiation device 1, and the rotational movement range of the arm unit 30 that is shown by an arc-shaped arrow M and segments positioned on both sides of the arrow M are displayed in the overhead omnidirectional image together with the subject H, the radiation detector 80, and the bed 3. The target position of the radiation-irradiation device 1 is calculated on the basis of the position of the radiation detector 80. That is, referring to the specifications of the already-known radiation-irradiation device 1, the position of the radiation-irradiation device 1 relative to the position of the radiation detector 80, at which a radiation image can be normally taken, is known. Accordingly, the position of the radiation-irradiation device 1 relative to the position of the radiation detector 80 may be set as a target position.

Returning to Fig. 11, the three positions of the radiation-irradiation device 1 shown in Fig. 11 are changed with time so as to be moved upward from the lower side in the plane of Fig. 11, that is, so as to gradually come close to the subject H. A movement path of the radiation-irradiation device 1 from the position, which is shown at the lowermost portion in Fig. 11, to a middle position in the vertical direction, that is, a movement path shown in Fig. 11 by a broken line T is a movement path along which the transport assistance is not performed, since obstacles are not present around the movement path, and an operator transports the radiation-irradiation device 1 on the basis of only one's own determination.

Since obstacles are present around the radiation-irradiation device 1 in a case in which the radiation-irradiation device 1 reaches a position shown in the middle in the vertical direction, the transport assistance in the "obstacle approach mode" having been set is started. Accordingly, first, information required for the transport assistance is displayed in the omnidirectional image shown in Fig. 9 or an omnidirectional image, in which the position of the radiation-irradiation device 1 is viewed from above, on the monitor 23. That is, for example, the appropriate movement path shown by the arrows Ar and the like, that is, a movement path that allows the radiation-irradiation device 1 not to collide or interfere with the obstacle B is displayed in the omnidirectional image shown in Fig. 9 as described above. Further, an appropriate movement path shown in Fig. 11 by an arrow U is displayed in the omnidirectional image in which the position of the radiation-irradiation device 1 is viewed from above. Alternatively, a message for the transport assistance using a voice of "go straight please as it is" or "turn right please" is notified by the speaker 18 shown in Fig. 6. If the radiation-irradiation device 1 deviates from the appropriate movement path in a case in which the transport assistance is performed, it is preferable that the notification of a warning indicating the fact of the deviation of the radiation-irradiation device 1 from the appropriate movement path is generated with a voice and the like generated from, for example, the speaker 18. Since the notification of a warning is generated, the operator can easily understand that the radiation-irradiation device 1 deviates from the appropriate movement path. Accordingly, the operator can instantly return the radiation-irradiation device 1 to the appropriate movement path.

In a case in which the radiation-irradiation device 1 is transported with reference to the notification of the appropriate movement path using the display or the voice, the radiation-irradiation device 1 finally reaches a target position shown at the uppermost portion in Fig. 11. The display of the overhead omnidirectional image of Fig. 15 shows a state in which the radiation-irradiation device 1 reaches a position shown at the uppermost portion in Fig. 11. In the example shown in Fig. 11, at a target position, the radiation detector 80 is present in the movable range of the arm unit 30 and obstacles are not present in the regions RA, RB, and RC of the work spaces having been set. Accordingly, it is preferable that the notification of the presence of the radiation detector 80 in the movable range of the arm unit 30 and the notification for ending the transport of the radiation-irradiation device 1 at that point of time are performed in this state. The notification can be performed by one or both of the notification using the display of the monitor 23 and the notification using the voice of the speaker 18. In a case in which the notification is performed, the operator can easily grasp that the radiation-irradiation device 1 is disposed at a position where the radiation image can be taken.

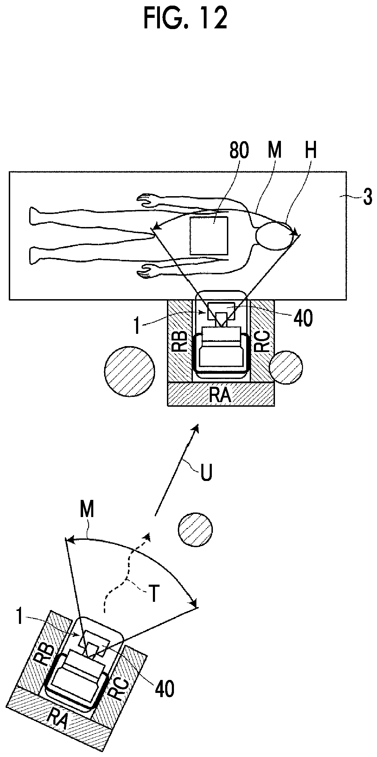

As long as the way of display of Figs. 12 to 14 to be described later is not particularly described, the way of display of Fig. 11 having described above is the same as that of Figs. 12 to 14 to be described later.

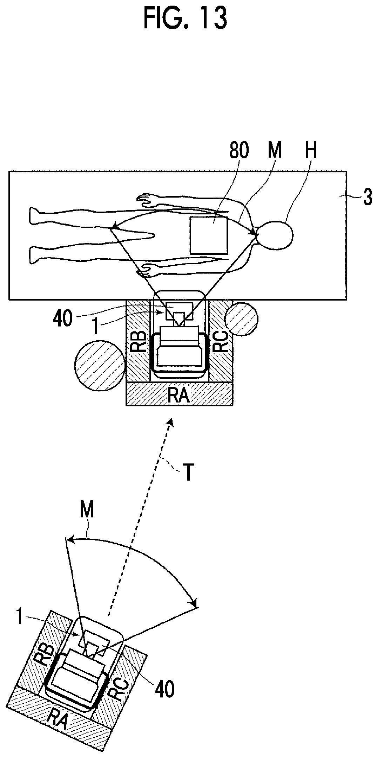

Next, the transport assistance in a case in which the presence circumstances of obstacles are different from those of the above-mentioned case will be described with reference to Figs. 12 and 13. In a case in which the radiation-irradiation device 1 reaches a target position, an obstacle is present in a part of the region RC among the regions RA, RB, and RC of the work spaces in an example shown in Fig. 12. The control unit 22 detects the fact that an obstacle is present in a part of the region RC on the basis of the omnidirectional image, and notifies an operator of the region in which the obstacle is present. The notification can be performed by one or both of the notification using the display of the monitor 23 and the notification using the voice of the speaker 18.

If the operator receives the notification in a case in which the operator transports the radiation-irradiation device 1 as shown by an arrow T of Fig. 12, the operator determines whether work for taking a radiation image cannot be performed completely due to the presence of the obstacle in the notified region RC or a radiation image can be taken even though the work is affected by the obstacle. In this case, if the operator determines that the latter is satisfied, the operator gives a command from the input unit 24 so that the transport assistance in the "obstacle approach mode" is started. In a case in which the control unit 22 receives the command, the control unit 22 starts the transport assistance. Accordingly, the appropriate movement path shown by an arrow U of Fig. 12 is displayed in an omnidirectional image in which, for example, the radiation-irradiation device 1 is viewed from above or an appropriate movement path shown by the arrows Ar is displayed in the omnidirectional image shown in Fig. 9 or 10, and a voice message representing the appropriate movement path is generated by the speaker 18.

In a case in which the radiation-irradiation device 1 reaches a target position, an obstacle is present in a part of the region RC among the regions RA, RB, and RC of the work spaces even in an example shown in Fig. 13 as in the example of Fig. 12. The control unit 22 detects the fact that an obstacle is present in a part of the region RC on the basis of the omnidirectional image, and notifies an operator of the region in which the obstacle is present. The notification can be performed by one or both of the notification using the display of the monitor 23 and the notification using the voice of the speaker 18.

If the operator receives the notification in a case in which the operator transports the radiation-irradiation device 1, the operator determines whether work for taking a radiation image cannot be performed completely due to the presence of the obstacle in the notified region RC or a radiation image can be taken even though the work is affected by the obstacle. In this case, the operator performs the above-mentioned determination in consideration of a direction in which the arm unit 30 extends.

In this case, if the operator determines that the former is satisfied, the operator does not input a command for performing the transport assistance. Accordingly, in this case, the operator transports the radiation-irradiation device 1 on the basis of only one's own determination, and makes the radiation-irradiation device 1 come close to the radiation detector 80 and the subject H along a movement path, which is shown in Fig. 13 by an arrow T, in a direction separate from a direction scheduled at the beginning. Since the omnidirectional image continues to be displayed even during the transport of the radiation-irradiation device 1, the operator can determine whether or not a radiation image can be taken even though the arm unit 30 extends in a case in which the radiation-irradiation device 1 reaches a target position. Here, a direction that allows an obstacle not to be present in a direction in which the arm unit 30 extends is selected as the direction separate from the originally scheduled direction.

In a case in which the radiation-irradiation device 1 reaches a target position, an obstacle is present in a part of the region RC among the regions RA, RB, and RC of the work spaces in the example shown in Fig. 13. In this case, the control unit 22 may detect the fact that an obstacle is present in a part of the region RC on the basis of the omnidirectional image, may determine a radiation-irradiation position in accordance with the region in which the obstacle is present, and may make the monitor 23 output a display to specify the determined position. That is, for example, the display of "Please irradiate the subject with radiation on the left side of the device" is shown on the monitor 23 in the example of Fig. 13. The operator views this display, and sets the arm unit 30 to, for example, a rotational movement position of the leftmost end of the movable range of Fig. 13 (the position of rotational movement about the rotational movement axis AX4). In this case, even though an obstacle is present in the region RC of the right work space, work for taking a radiation image can be performed using the region RB of the left work space without being affected by the obstacle.

Determining whether work for taking a radiation image cannot be performed completely due to the presence of the obstacle or a radiation image can be taken even though the work is affected by the obstacle and making the radiation-irradiation device 1 come close to the radiation detector 80 and the subject H in a direction separate from a direction scheduled at the beginning, which have been described above, may be performed on the transport assistance in the "target position approach mode" to be described below.

Further, in a case in which the transport assistance is not performed since an obstacle is not detected during the setting of the transport assistance in the above-mentioned "obstacle approach mode", the transport assistance in the "target position approach mode" may be performed when the current position of the radiation-irradiation device 1 comes close to a target position by a predetermined distance.

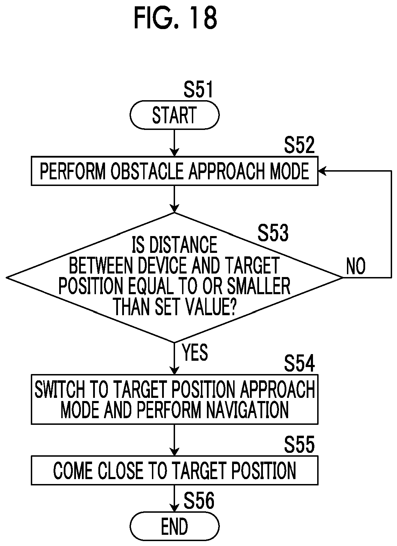

The flow of processing in this case will be described with reference to a flow chart of Fig. 18. This processing is started in Step S51, and the obstacle approach mode is performed at the beginning (Step S52). While the obstacle approach mode is performed, it is determined whether or not a distance between the radiation-irradiation device 1 and a target position is equal to or smaller than a set value in Step S53. For example, the set value is set to about 3 m. If the determination of "NO" is made in Step S53, the processing of Step S52 and the processing of Step S53 are repeated.

If the determination of "YES" is made in Step S53, a mode is switched to the target position approach mode from the obstacle approach mode and the transport assistance is performed in Step S54. The radiation-irradiation device 1 comes close to a target position as it is (Step S55), and the processing ends in Step S56. Accordingly, since the radiation-irradiation device 1 does not come close to an obstacle, the guide assistance is not performed. For this reason, a situation in which the radiation-irradiation device 1 collides with the bed and the like can be prevented.

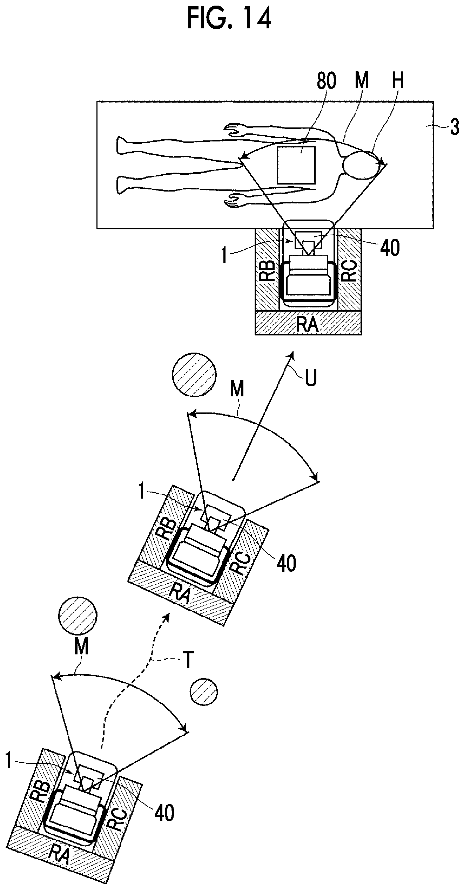

Next, the transport assistance of the radiation-irradiation device 1 performed in the "target position approach mode" will be described with reference to Fig. 14. The "target position approach mode" is a mode in which the transport assistance is started regardless of whether or not an obstacle is present near the radiation-irradiation device 1 in a case in which the radiation-irradiation device 1 comes close to a target position and is a mode suitable for a case in which the forward visibility of the radiation-irradiation device 1 is good. That is, since freedom in operator's work deteriorates in a case in which the transport assistance using the display of an image or a voice is performed even though an operator easily transport the radiation-irradiation device 1 due to good forward visibility, a feeling of restriction is given to the operator. Accordingly, it is preferable that the transport assistance is not performed while the radiation-irradiation device 1 is far from the target position.

Fig. 14 shows an example of a change in the transport position of the radiation-irradiation device 1 in this mode. Fig. 14 shows a state in which the position of the radiation-irradiation device 1 is viewed from above. However, as described above, an omnidirectional image shown in this state is also displayed on the monitor 23 instead of the omnidirectional images shown in the states of Figs. 9 and 10. In this case, two omnidirectional images are switched and displayed in a case in which, for example, the operator inputs a command for switching the display state of an omnidirectional image from the input unit 24. For example, the switching of the display image is performed to allow images shown in the states of Figs. 9 and 10 to be displayed while the radiation-irradiation device 1 is relatively far from the target position and to allows an image, which shows the vicinity of the radiation detector 80 as shown in Fig. 15, to be displayed in a case in which the radiation-irradiation device 1 comes close to the target position. Not an image that is formed through the above-mentioned reconstruction but an image taken by one camera may be used as the image that shows the vicinity of the radiation detector 80. Further, in a case in which only the images of the states of Figs. 9 and 10 are displayed without the above-mentioned switching and display of the omnidirectional images, only one camera, which takes images of the circumstances on the front side of the radiation-irradiation device 1, may be used and the taken images may be displayed.

Returning to Fig. 14, three positions of the radiation-irradiation device 1 shown in Fig. 14 are changed with time so as to be moved upward from the lower side, that is, so as to come close to the subject H. A movement path of the radiation-irradiation device 1 from the position, which is shown at the lowermost portion in Fig. 14, to a middle position in the vertical direction, that is, a movement path shown in Fig. 14 by a broken line T is a path along which the transport assistance is not performed, since the radiation-irradiation device 1 is not close to a target position, and an operator transports the radiation-irradiation device 1 on the basis of only one's own thought.

In a case in which the radiation-irradiation device 1 reaches a position shown in the middle in the vertical direction, that is, a position that is away from the target position of the radiation-irradiation device 1 by an appropriate distance determined in advance, the transport assistance in the "target position approach mode" having been set is started. Accordingly, first, information required for the transport assistance is displayed in the omnidirectional image shown in Fig. 9 or an omnidirectional image, in which the position of the radiation-irradiation device 1 is viewed from above, on the monitor 23. That is, for example, the appropriate movement path shown by the arrows Ar and the like, that is, a movement path that allows the radiation-irradiation device 1 not to collide or interfere with the obstacle B is displayed in the omnidirectional image shown in Fig. 9 as described above. Further, an appropriate movement path shown in Fig. 14 by an arrow U is displayed in the omnidirectional image in which the position of the radiation-irradiation device 1 is viewed from above. Alternatively, a message for the transport assistance using a voice of "go straight please as it is" or "turn right please" is notified by the speaker 18 shown in Fig. 6. Since the operator may be annoyed in a case in which the message for the transport assistance using the voice is unnecessary, the message for the transport assistance using the voice may be adapted not to be notified until the radiation-irradiation device 1 comes close to a target position to some extent. Further, if the radiation-irradiation device 1 deviates from the appropriate movement path in a case in which the transport assistance is performed, it is preferable that the notification of a warning indicating the fact of the deviation of the radiation-irradiation device 1 from the appropriate movement path is generated with a voice and the like generated from, for example, the speaker 18.

In a case in which the radiation-irradiation device 1 is transported with reference to the notification of the appropriate movement path using the display or the voice, the radiation-irradiation device 1 finally reaches a target position shown at the uppermost portion in Fig. 14. In the example shown in Fig. 14, at the target position, the radiation detector 80 is present in the movable range of the arm unit 30 and obstacles are not present in the regions RA, RB, and RC of the work spaces having been set. Accordingly, it is preferable that the notification of the presence of the radiation detector 80 in the movable range of the arm unit 30 and the notification for ending the transport of the radiation-irradiation device 1 at that point of time are performed in this state. The notification can be performed by one or both of the notification using the display of the monitor 23 and the notification using the voice of the speaker 18.

In a case in which the radiation-irradiation device 1 reaches a target position after the transport assistance of the radiation-irradiation device 1 is performed in the "obstacle approach mode" or the "target position approach mode" having been described above, a radiation image is taken by the radiation-irradiation device 1 present at the position. The taking of the radiation image is generally performed by the following procedure.

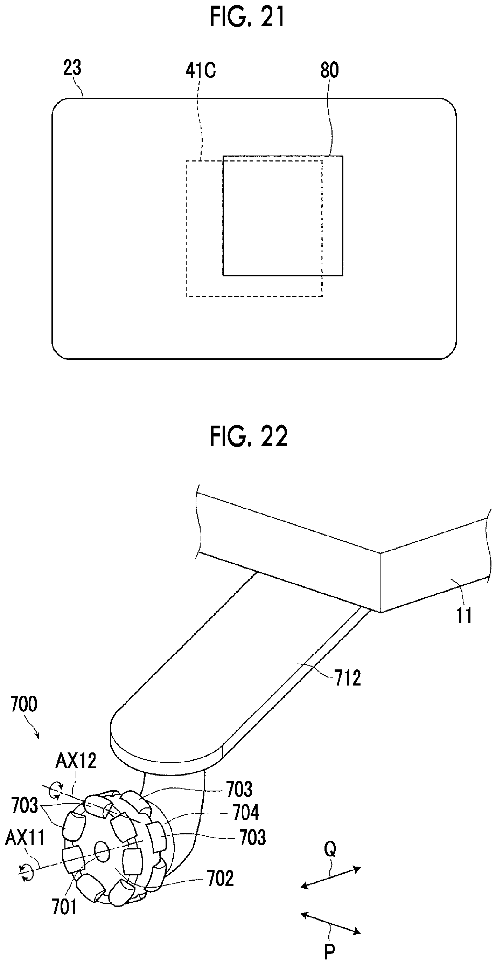

First, the rotation of the wheels of the wheel parts 12 is locked so that the radiation-irradiation device 1 is fixed at the target position. Next, an image display mode of the monitor 23 is automatically or manually switched from a mode that displays a reconstruct omnidirectional image to a mode that displays the radiation detector 80 and a radiation-irradiation field around the subject H. An image, which is displayed in the latter display mode, is generally taken by a camera (not shown) that is mounted on the radiation source unit 40. Fig. 21 shows an example of the image that is displayed in the latter display mode. In this embodiment, the position of a collimator 41C of the radiation source unit 40 is displayed together with the position of the radiation detector 80. For example, the markers 84A to 84D, which are adapted to transmit identification information by radio as described above, are detected by a receiver (not shown) provided on the radiation-irradiation device 1, and the position of the radiation detector 80 is detected on the basis of the detection information of the receiver. Further, the position of the collimator 41C is calculated on the basis of, for example, the specifications of the radiation source unit 40 and the detected position of the radiation source unit 40 (which can be detected from the state of the arm unit 30). The display of the positions of the radiation detector 80 and the collimator 41C, which are obtained in this way, is overlaid on an optical image that is taken by the camera.

An operator operates the arm unit 30 while observing the image, and shifts the radiation detector 80 to a state in which the power source is turned on from a previous sleeve state in a case in which the radiation detector 80 is seen in the image displayed on the monitor 23. The operator continues to observe the image. Finally, the operator visually confirms the subject H and the circumstances around the subject H, and operates the arm unit 30 so that, for example, the center of the radiation detector 80 and the center of the collimator 41C correspond to each other. Then, the operator shifts the radiation detector 80 to an imaging-ready state from a previous standby state.

Next, a series of operations, such as radiation exposure, electric charges accumulation of the radiation detector 80, the reading of a radiation image corresponding to the accumulated electric charges, and the transmission of image data, are performed, and the taking of the radiation image ends.

Further, in a case in which the taking of a radiation image ends, the display mode of the monitor 23 is switched to an omnidirectional image display mode by an automatic or manual operation and the radiation-irradiation device 1 is in a state in which the radiation-irradiation device 1 can be transported to another place.

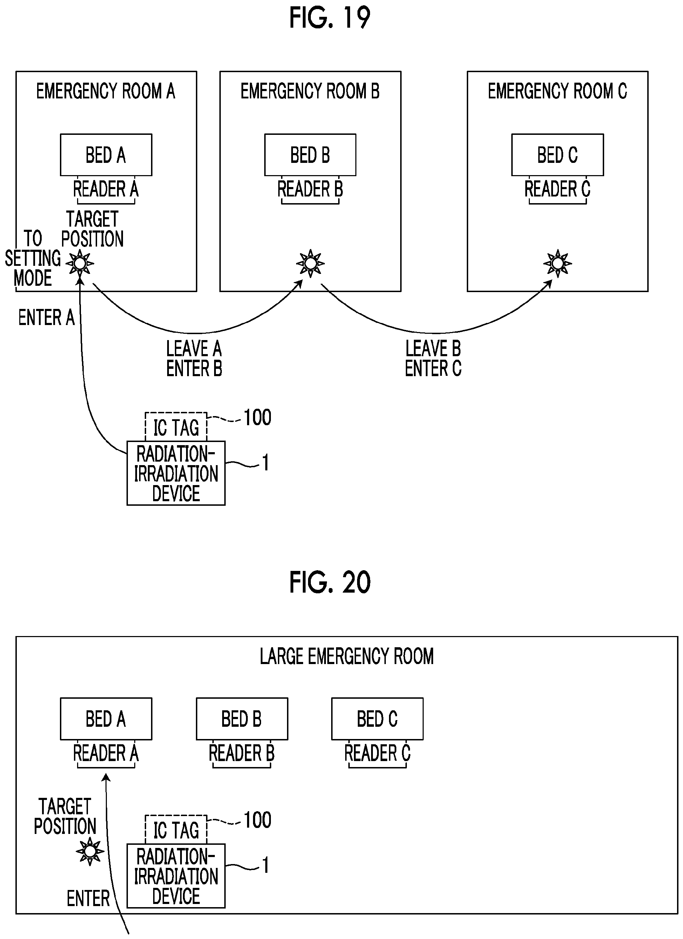

Next, the timing of the setting of the above-mentioned target position will be described in detail with reference to Figs. 19 and 20. A case in which a plurality of rooms where the radiation-irradiation device 1 is to be used are provided and target positions are to be individually set for beds provided in the respective rooms and a case in which only one room where the radiation-irradiation device 1 is to be used is provided and target positions are to be individually set for a plurality of beds provided in the room are considered in regard to the setting of the target position.

Fig. 19 is a diagram schematically illustrating the former case. In this example, three rooms, that is, an emergency room A, an emergency room B, and an emergency room C are provided and a bed A, a bed B, and a bed C are placed in the emergency room A, the emergency room B, and the emergency room C, respectively. Further, the above-mentioned readers A, B, and C are disposed close to the beds A, B, and C, respectively. Furthermore, the above-mentioned IC tag 100 is mounted on the radiation-irradiation device 1.

As shown in Fig. 19, the radiation-irradiation device 1 enters the respective rooms in the order of, for example, the emergency room A, the emergency room B, and the emergency room C and sets a target position in each room. First, in a case in which the radiation-irradiation device 1 enters the emergency room A, the radiation-irradiation device 1 is shifted to a target position-setting mode. Further, in a case in which the radiation-irradiation device 1 comes close to the reader A, the reader A reads the IC tag 100. In this case, a place in which the reader A is disposed is a target place for the setting of a target position.

In a case in which a target position is set in the emergency room A as described above, the radiation-irradiation device 1 is sequentially transported into the emergency room B and the emergency room C and a target position is set in each room as in the emergency room A.

The setting of a reading distance of the IC tag 100 (generally about 10 m) is important. That is, in a case in which an omnidirectional image is acquired at the read position by a camera mounted on the radiation-irradiation device 1, it is preferable that the bed is included in the omnidirectional image. That is, in a case in which the bed is not included in the omnidirectional image, a target position cannot be set. Further, combinations of the readers A, B, and C and the IC tag 100 are registered in the radiation-irradiation device 1 in advance. Furthermore, the readers A, B, and C do not necessarily need to be disposed close to the beds, and may be disposed other appropriate places (for example, walls and the like) in the rooms. A method in which an AP (access point) is built in the bed and the radiation-irradiation device 1 receives beacon signals regularly transmitted from the AP can also be applied as other communication between the radiation-irradiation device 1 and the room. Considering that peripheral devices are placed in an emergency room or drip poles and the like often serve as obstacles, it can be said that this communication method is particularly preferable.

Fig. 20 is a diagram schematically illustrating the case in which only one room where the radiation-irradiation device 1 is to be used is provided and target positions are to be individually set for a plurality of beds provided in the room. In this case, since the plurality of beds are provided in the emergency room, it is difficult to know which bed a target position is to be set for not only before the radiation-irradiation device 1 enters the emergency room but also immediately after the radiation-irradiation device 1 enters the emergency room. Accordingly, the readers A, B, and C are disposed on the beds A, B, and C, respectively; each reader can read the IC tag 100 in a case in which the radiation-irradiation device 1 comes close to each reader; and the radiation-irradiation device 1 is shifted to the target position-setting mode.

In this case, an IC tag readable distance is appropriately set in each of the readers A, B, and C. If the beds A, B, and C are very close to each other, there is a case where a plurality of readers may perform reading. To prepare for such a case, it is preferable that the monitor 23 of the radiation-irradiation device 1 displays reading performed by the plurality of readers to allow an operator to select a bed for which a target position is to be set.

In regard to a wheel part that is applied to the radiation-irradiation device 1, an example of the wheel part other than the above-mentioned caster-shaped wheel part 12 will be described here. A wheel part shown in Fig. 22 is composed of, for example, OMNI WHEEL (registered trademark). Fig. 22 shows a state in which the OMNI WHEEL 700 is mounted on the base 11 of the radiation-irradiation device 1 shown in Fig. 1 through a leg holding portion 712 as an example.

The OMNI WHEEL 700 is one of omni directionally moving wheels, and includes a rotating body 702 that is mounted on an axle 701 and is rotatable about a rotation axis AX11 in a normal direction and a reverse direction, and a plurality of rollers 703 that are mounted on the outer peripheral portion of the rotating body 702. For example, a barrel-shaped roller is applied as the roller 703.

In this example, seven rollers 703 are mounted on each of left and right sides of the rotating body 702, that is, a total of fourteen rollers 703 are mounted on the rotating body 702. Each of the seven rollers 703, which are mounted on one side of the left and right sides of the rotating body, is mounted on the rotating body 702 so as to be rotatable about a rotation axis AX12, which extends in a tangential direction of one circle coaxial with the rotation axis AX11, in a normal direction and a reverse direction. The same applies to the seven rollers 703 that are mounted on the other side of the left and right sides of the rotating body. Further, the seven rollers 703, which are mounted on one side of the left and right sides of the rotating body, are disposed at positions that face gaps between the seven rollers 703 that are mounted on the other side of the left and right sides of the rotating body. The OMNI WHEEL 700 having the above-mentioned structure is mounted on each leg holding portion 712 through a bearing part 704 receiving the axle 701.

In the case of the OMNI WHEEL 700, the rotating body 702 and the fourteen rollers 703 form one rotating wheel. That is, in a case in which a force acting in the direction of an arrow P of Fig. 22 is applied to the radiation-irradiation device including the leg holding portions 712, each wheel, which includes the rotating body 702 and the rollers 703, rotates about the rotation axis AX11 while the fourteen rollers 703 serve as the outer peripheral surface of each wheel. Accordingly, the movement of the legs 12, that is, the radiation-irradiation device in the direction of the arrow P is facilitated. Further, in a case in which a force acting in the direction of an arrow Q of Fig. 22 is applied to the radiation-irradiation device including the leg holding portions 712, each of the grounded rollers 703 rotates about the rotation axis AX12. Accordingly, the movement of the leg holding portions 712, that is, the radiation-irradiation device in the direction of the arrow Q is facilitated.

For example, a Mecanum wheel disclosed in JP2013-081659A can also be applied as the omni directionally moving wheel other than the above-mentioned OMNI WHEEL 700.

### Explanation of References

1: radiation-irradiation device
2: device-placement surface
3: bed
10: leg unit
11: base
12: wheel part
18: speaker
20: body unit
21: housing
22: control unit
23: monitor
24: input unit
28A to 28D: camera
30: arm unit
31: first arm
32: second arm
40: radiation source unit
50: raising/lowering mechanism
80: radiation detector
84A to 84D: marker of radiation detector
100: IC tag
700: OMNI WHEEL

## Claims

1. A transport assisting method for a radiation-irradiation device assisting a user to transport a radiation-irradiation device (1), on which a radiation source (40), which is configured for irradiating a radiation image recording medium (80), which is disposed under a subject (H) on a bed, with radiation, and a movable radiation source holding unit (30), which is configured for holding the radiation source (40) so as to allow the position of the radiation source to be changeable, are mounted, wherein the radiation-irradiation device is capable of being transported by the user to a target position, the transport assisting method comprising:
setting the target position of the radiation-irradiation device (1), where the radiation image recording medium (80) is in a movable range of the radiation source holding unit (30) such that the position of the radiation source (40) is capable of being changed by the radiation source holding unit and the radiation source is capable of being disposed at a radiation-irradiation position where a radiation image is capable of being taken, and where a work space, which is set to a size that allows at least one person to easily pass, is capable of being secured around the radiation-irradiation device (1);
detecting a current position of the radiation-irradiation device;
detecting a position of an obstacle that obstructs the transport of the radiation-irradiation device;
and notifying the user of the transport assistance of the movement path of the radiation-irradiation device to the target position on the basis of the target position, the current position, and the position of the obstacle.

2. The transport assisting method for a radiation-irradiation device according to claim 1, wherein the notifying is started in a case in which the radiation-irradiation device approaches the target position by a predetermined distance, which is shorter than a distance to the target position at the time of setting of the target position, after the setting of the target position.

3. The transport assisting method for a radiation-irradiation device according to claim 1, wherein the notifying is started in a case in which the radiation-irradiation device approaches the obstacle by a predetermined distance after the setting of the target position.

4. The transport assisting method for a radiation-irradiation device according to any one of claims 1 to 3, wherein in a case in which the radiation-irradiation device is positioned at the target position, the work space is secured on at least a right side of the radiation-irradiation device in a case in which the radiation-irradiation position of the radiation source is present on the right side of the radiation-irradiation device and the work space is secured on at least a left side of the radiation-irradiation device in a case in which the radiation-irradiation position of the radiation source is present on the left side of the radiation-irradiation device.

5. The transport assisting method for a radiation-irradiation device according to any one of claims 1 to 4, wherein a position of the radiation image recording medium, which is used to set the target position, is detected on the basis of an image that is reconstructed from images taken by a plurality of cameras mounted on the radiation-irradiation device and/or an image that is taken by one camera.

6. The transport assisting method for a radiation-irradiation device according to any one of claims 1 to 5, wherein a position of the radiation image recording medium, which is used to set the target position, is detected on the basis of a radio signal that is transmitted from the radiation image recording medium.

7. The transport assisting method for a radiation-irradiation device according to any one of claims 1 to 6, wherein a position of the radiation image recording medium, which is used to set the target position, is acquired from an order for taking a radiation image.

8. The transport assisting method for a radiation-irradiation device according to any one of claims 1 to 7, wherein the current position of the radiation-irradiation device is detected on the basis of an image that is reconstructed from images taken by a plurality of cameras mounted on the radiation-irradiation device and/or an image that is taken by one camera.

9. The transport assisting method for a radiation-irradiation device according to any one of claims 1 to 8, wherein the position of the obstacle is detected on the basis of an image that is reconstructed from images taken by a plurality of cameras mounted on the radiation-irradiation device and/or an image that is taken by one camera.

10. The transport assisting method for a radiation-irradiation device according to any one of claims 1 to 9, wherein the setting of the target position is performed under conditions where the radiation source is capable of being disposed at the radiation-irradiation position where a radiation image is capable of being taken with respect to the radiation image recording medium in a movable range where the position of the radiation source is capable of being changed by the radiation source holding unit, the work space is capable of being secured around the radiation-irradiation device, and the obstacle is not present in a movable range of the radiation source holding unit.

11. The transport assisting method for a radiation-irradiation device according to any one of claims 1 to 9, wherein the setting of the target position is performed under conditions where the radiation source is capable of being disposed at the radiation-irradiation position where a radiation image is capable of being taken with respect to the radiation image recording medium in a movable range where the position of the radiation source is capable of being changed by the radiation source holding unit, the work space is capable of being secured around the radiation-irradiation device, and whether or not a radiation image is not capable of being taken due to the presence of the obstacle in a case in which the obstacle is present in a movable range of the radiation source holding unit.

12. The transport assisting method for a radiation-irradiation device according to any one of claims 1 to 11, wherein the notifying is performed using an image that is displayed on display means for displaying an image.

13. The transport assisting method for a radiation-irradiation device according to claim 12, wherein the image, which is displayed on the display means as the notifying, includes a display that shows a path to the target position from the current position of the radiation-irradiation device.

14. The transport assisting method for a radiation-irradiation device according to claim 12 or 13, wherein the image, which is displayed on the display means as the notifying, includes an image that shows a field of view on a front side of the radiation-irradiation device in a transport direction.

15. The transport assisting method for a radiation-irradiation device according to claim 12 or 13, wherein the image, which is displayed on the display means as the notifying, includes an image obtained by viewing the radiation-irradiation device from above.

16. The transport assisting method for a radiation-irradiation device according to any one of claims 1 to 11, wherein the notifying is performed using a voice.

17. The transport assisting method for a radiation-irradiation device according to claim 16, wherein the notifying using the voice is to notify the user of a transport direction with respect to the transport direction of the radiation-irradiation device at a point of time in a case in which the notifying is performed.

18. The transport assisting method for a radiation-irradiation device according to any one of claims 1 to 17, wherein in a case in which the radiation-irradiation device deviates from an appropriate movement path based on the notifying during the transport of the radiation-irradiation device, a notification of a warning indicating a fact of the deviation of the radiation-irradiation device is generated.

19. The transport assisting method for a radiation-irradiation device according to any one of claims 1 to 18, wherein in a case in which the radiation-irradiation device arrives at the target position, a notification to the user of the arrival of the radiation-irradiation device is generated.

20. A transport assisting device for a radiation-irradiation device assisting a user to transport a radiation-irradiation device (1), on which a radiation source (40), which is configured for irradiating a radiation image recording medium (80), which is disposed under a subject (H) on a bed, with radiation, and a movable radiation source holding unit (30), which is configured for holding the radiation source (40) so as to allow the position of the radiation source to be changeable, are mounted, wherein the radiation-irradiation device is capable of being transported by the user to a target position, the transport assisting device comprising:
target position setting means for setting the target position of the radiation-irradiation device (1), where the radiation image recording medium (80) is in a movable range of the radiation source holding unit (30) such that the position of the radiation source (40) is capable of being changed by the radiation source holding unit and the radiation source is capable of being disposed at a radiation-irradiation position where a radiation image is capable of being taken, and where a work space, which is set to a size that allows at least one person to easily pass, is capable of being secured around the radiation-irradiation device (1);
current position detecting means for detecting a current position of the radiation-irradiation device;
obstacle position detection means for detecting a position of an obstacle that obstructs the transport of the radiation-irradiation device;
and notification means for notifying the user of the transport assistance of the movement path of the radiation-irradiation device to the target position on the basis of the target position, the current position, and the position of the obstacle.

21. A radiation-irradiation device, on which a radiation source, which is configured for irradiating a radiation image recording medium with radiation, and a movable radiation source holding unit, which is configured for holding the radiation source so as to allow the position of the radiation source to be changeable, are mounted, wherein the radiation-irradiation device is capable of being transported by the user to a target position, the radiation-irradiation device comprising:
The transport assisting device according to claim 20.

## Patentansprüche

1. Transportunterstützungsverfahren für eine Bestrahlungsvorrichtung, um einen Benutzer beim Transportieren einer Bestrahlungsvorrichtung (1) zu unterstützen, an der eine Strahlungsquelle (40), konfiguriert zum Bestrahlen eines Strahlungsbild-Aufzeichnungsmediums (80), das sich unter einem Subjekt (H) auf einem Bett befindet, mit Strahlung, und eine bewegliche Strahlungsquellen-Halteeinheit (30), konfiguriert zum Halten der Strahlungsquelle (40), um die Position der Strahlungsquelle änderbar zu machen, angebracht sind, wobei die Bestrahlungsvorrichtung in der Lage ist, von dem Benutzer zu einer Zielposition transportiert zu werden, umfassend:
Einstellen der Zielposition der Bestrahlungsvorrichtung (1), wobei das Strahlungsbild-Aufzeichnungsmedium (80) sich in einem bewegbaren Bereich der Strahlungsquellen-Halteeinheit (30) befindet, so dass die Position der Strahlungsquelle (40) von der Strahlungsquellen-Halteeinheit geändert werden kann, und die Strahlungsquelle in der Lage ist, an einer Bestrahlungsposition angeordnet zu werden, an welcher ein Strahlungsbild aufgenommen werden kann, und wobei ein Arbeitsraum mit einer so eingestellten Größe, dass mindestens eine Person leicht passieren kann, um die Bestrahlungsvorrichtung (1) herum gewährleistet werden kann;
Detektieren einer laufenden Position der Bestrahlungsvorrichtung;
Detektieren einer Position eines Hindernisses, welches den Transport der Bestrahlungsvorrichtung behindert; und
dem Benutzer der Transportunterstützung wird der Bewegungsweg der Bestrahlungsvorrichtung zu der Zielposition gemeldet auf der Grundlage der Zielposition, der laufenden Position und der Position des Hindernisses.

2. Verfahren nach Anspruch 1, bei dem das Melden dann begonnen wird, wenn sich die Bestrahlungsvorrichtung der Zielposition um eine vorbestimmte Distanz nähert, welche kürzer ist als eine Distanz zu der Zielposition zu dem Zeitpunkt des Einstellens der Zielposition, nachdem die Zielposition eingestellt wurde.

3. Verfahren nach Anspruch 1, bei dem das Melden dann gestartet wird, wenn die Bestrahlungsvorrichtung sich dem Hindernis um eine vorbestimmte Distanz nach Einstellen der Zielposition nähert.

4. Verfahren nach einem der Ansprüche 1 bis 3, bei dem dann, wenn die Bestrahlungsvorrichtung an der Zielposition positioniert ist, der Arbeitsraum zumindest auf einer rechten Seite der Strahlungsvorrichtung dann eingerichtet wird, wenn die Bestrahlungsposition der Bestrahlungsquelle sich auf der rechten Seite der Strahlungsvorrichtung befindet, und der Arbeitsraum auf zumindest einer linken Seite der Strahlungsvorrichtung dann eingerichtet wird, wenn die Bestrahlungsposition der Strahlungsquelle sich auf der linken Seite der Bestrahlungsvorrichtung befindet.

5. Verfahren nach einem der Ansprüche 1 bis 4, bei dem eine Position des Strahlungsbild-Aufzeichnungsmediums, das zum Einrichten der Zielposition verwendet wird, auf der Grundlage eines Bilds nachgewiesen wird, das rekonstruiert wird aus Bildern, die von mehreren Kameras aufgenommen werden, die an der Strahlungsvorrichtung angebracht sind, und/oder aufgrund eines von einer Kamera aufgenommenen Bilds nachgewiesen wird.

6. Verfahren nach einem der Ansprüche 1 bis 5, bei dem eine Position des Strahlungsbild-Aufzeichnungsmediums, das zum Einrichten der Zielposition verwendet wird, nachgewiesen wird auf der Grundlage eines Funksignals, welches von dem Strahlungsbild-Aufzeichnungsmedium gesendet wird.

7. Verfahren nach einem der Ansprüche 1 bis 6, bei dem eine Position des Strahlungsbild-Aufzeichnungsmediums, das zum Einrichten der Zielposition verwendet wird, von einem Auftrag zum Aufnehmen eines Strahlungsbilds entnommen wird.

8. Verfahren nach einem der Ansprüche 1 bis 7, bei dem die laufende Position der Bestrahlungsvorrichtung nachgewiesen wird auf der Grundlage eines Bilds, welches aus Bildern rekonstruiert ist, die von mehreren an der Bestrahlungsvorrichtung angebrachten Kameras aufgenommen werden, und/oder auf der Grundlage eines Bilds, das von einer Kamera aufgenommen wird.

9. Verfahren nach einem der Ansprüche 1 bis 8, bei dem die Position des Hindernisses auf der Grundlage eines Bilds nachgewiesen wird, das aus Bildern rekonstruiert ist, die von mehreren Kameras aufgenommen wurden, die an der Bestrahlungsvorrichtung angebracht sind, und/oder anhand eines Bilds nachgewiesen wird, das von einer Kamera aufgenommen wird.

10. Verfahren nach einem der Ansprüche 1 bis 9, bei dem die Einstellung der Zielposition unter Bedingungen ausgeführt wird, nach denen die Strahlungsquelle in der Lage ist, an der Bestrahlungsposition angeordnet zu werden, wo ein Strahlungsbild bezüglich des Strahlungsbild-Aufzeichnungsmediums aufgenommen werden kann in einem beweglichen Bereich, in welchem die Position der Strahlungsquelle durch die Strahlungsquellen-Halteeinheit geändert werden kann, der Arbeitsraum um die Bestrahlungsvorrichtung herum gewährleistet werden kann und sich das Hindernis nicht in einem beweglichen Bereich der Strahlungsquellen-Halteeinheit befindet.

11. Verfahren nach einem der Ansprüche 1 bis 9, bei dem das Einstellen der Zielposition ausgeführt wird unter Bedingungen, nach denen die Strahlungsquelle in der Lage ist, an der Bestrahlungsposition angeordnet zu werden, wo ein Strahlungsbild in Bezug auf das Strahlungsbild-Aufzeichnungsmedium in einem beweglichen Bereich aufgenommen werden kann, wo die Position der Strahlungsquelle von der Strahlungsquellen-Halteeinheit geändert werden kann, der Arbeitsraum um die Bestrahlungsvorrichtung herum gewährleistet werden kann, und abhängig davon, ob ein Strahlungsbild nicht aufgenommen werden kann aufgrund des Vorhandenseins des Hindernisses in dem Fall, dass sich das Hindernis in einem beweglichen Bereich der Strahlungsquellen-Halteeinheit befindet.

12. Verfahren nach einem der Ansprüche 1 bis 11, bei dem das Melden unter Verwendung eines Bilds erfolgt, das auf einer Anzeigeeinrichtung zum Anzeigen eines Bilds angezeigt wird.

13. Verfahren nach Anspruch 12, bei dem das auf der Anzeigeeinrichtung zum Melden angezeigte Bild eine Anzeige beinhaltet, die einen Weg zu der Zielposition ausgehend von der laufenden Position der Bestrahlungsvorrichtung zeigt.

14. Verfahren nach Anspruch 12 oder 13, bei dem das Bild, das auf der Anzeigeeinrichtung zum Melden angezeigt wird, ein Bild enthält, welches ein Gesichtsfeld einer Frontseite der Bestrahlungsvorrichtung in Transportrichtung zeigt.

15. Verfahren nach Anspruch 12 oder 13, bei dem das Bild, das auf der Anzeigeeinrichtung zum Melden angezeigt wird, ein Bild enthält, das gewonnen ist durch Betrachten der Bestrahlungsvorrichtung von oben.

16. Verfahren nach einem der Ansprüche 1 bis 11, bei dem das Melden durch eine Stimme erfolgt.

17. Verfahren nach Anspruch 16, bei dem das Melden mittels der Stimme dazu dient, den Benutzer über eine Transportrichtung bezüglich der Transportrichtung der Strahlungsvorrichtung zu einem Zeitpunkt zu informieren, zu dem das Melden erfolgt.

18. Verfahren nach einem der Ansprüche 1 bis 17, bei dem dann, wenn die Bestrahlungsvorrichtung von einem angemessenen Bewegungsweg abweicht entsprechend der Meldung während des Transports der Bestrahlungsvorrichtung, eine Warnmeldung erfolgt, die den Umstand angibt, dass eine Abweichung der Bestrahlungsvorrichtung vorliegt.

19. Verfahren nach einem der Ansprüche 1 bis 18, bei dem, wenn die Bestrahlungsvorrichtung an der Zielposition ankommt, eine Meldung erzeugt wird, die den Benutzer über die Ankunft der Bestrahlungsvorrichtung informiert.

20. Transportunterstützungsvorrichtung für eine Bestrahlungsvorrichtung, um einen Benutzer beim Transportieren einer Bestrahlungsvorrichtung (1) zu unterstützen, an der eine Strahlungsquelle (40), konfiguriert zum Bestrahlen eines Strahlungsbild-Aufzeichnungsmediums (80), das sich unter einem Subjekt (H) auf einem Bett befindet, mit Strahlung, und eine bewegliche Strahlungsquellen-Halteeinheit (30), konfiguriert zum Halten der Strahlungsquelle (40), um die Position der Strahlungsquelle änderbar zu machen, angebracht sind, wobei die Bestrahlungsvorrichtung in der Lage ist, von dem Benutzer zu einer Zielposition transportiert zu werden, aufweist:
eine Zielpositions-Einstelleinrichtung zum Einstellen der Zielposition der Bestrahlungsvorrichtung (1), wobei das Strahlungsbild-Aufzeichnungsmedium (80) sich in einem Bewegungsbereich der Strahlungsquellen-Halteeinheit (30) befindet, so dass die Position der Strahlungsquelle (40) durch die Strahlungsquellen-Halteeinheit geändert werden kann, und die Strahlungsquelle in der Lage ist, an einer Bestrahlungsposition angeordnet zu werden, wo ein Strahlungsbild aufgenommen werden kann, und wobei ein Arbeitsraum, der auf eine Größe eingestellt ist, die es mindestens einer Person ermöglicht, leicht zu passieren, um die Bestrahlungsvorrichtung (1) herum eingerichtet werden kann;
eine Laufendposition-Nachweiseinrichtung zum Nachweisen einer laufenden Position der Bestrahlungsvorrichtung;
eine Hindernisposition-Nachweiseinrichtung zum Nachweisen einer Position eines Hindernisses, welches den Transport der Bestrahlungsvorrichtung behindert; und
eine Meldeeinrichtung, um dem Benutzer der Transportunterstützung über den Bewegungsweg der Bestrahlungsvorrichtung zu der Zielposition auf der Grundlage der Zielposition, der laufenden Position und der Position des Hindernisses zu informieren.

21. Bestrahlungsvorrichtung, an der eine Strahlungsquelle, konfiguriert zum Bestrahlen eines Strahlungsbild-Aufzeichnungsmediums mit Strahlung und eine bewegliche Strahlungsquellen-Halteeinheit, konfiguriert zum Halten der Strahlungsquelle derart, dass die Position der Strahlungsquelle änderbar ist, angebracht sind, wobei die Bestrahlungsvorrichtung in der Lage ist, von dem Benutzer zu einer Zielposition transportiert zu werden, wobei die Bestrahlungsvorrichtung die Transportunterstützungsvorrichtung nach Anspruch 20 enthält.

## Revendications

1. Procédé d'assistance de transport pour un dispositif d'irradiation par rayonnement assistant un utilisateur pour transporter un dispositif d'irradiation par rayonnement (1), sur lequel sont montés une source de rayonnement (40), laquelle est configurée pour irradier avec un rayonnement un support d'enregistrement d'image de rayonnement (80), lequel est disposé sous un sujet (H) sur un lit, et une unité de tenue de source de rayonnement pouvant être déplacée (30), laquelle est configurée pour tenir la source de rayonnement (40) de manière à permettre de modifier la position de la source de rayonnement, dans lequel le dispositif d'irradiation par rayonnement peut être transporté par l'utilisateur jusqu'à une position cible, le procédé d'assistance de transport comprenant les étapes suivantes :
régler la position cible du dispositif d'irradiation par rayonnement (1), où le support d'enregistrement d'image de rayonnement (80) se trouve dans une plage pouvant être déplacée de l'unité de tenue de source de rayonnement (30) de telle sorte que la position de la source de rayonnement (40) peut être modifiée par l'unité de tenue de source de rayonnement et que la source de rayonnement peut être disposée sur une position d'irradiation par rayonnement où une image de rayonnement peut être prise, et où un espace de travail, lequel est réglé sur une taille permettant à au moins une personne de passer facilement, peut être sécurisé autour du dispositif d'irradiation par rayonnement (1) ;
détecter une position actuelle du dispositif d'irradiation par rayonnement ;
détecter une position d'un d'obstacle, lequel obstrue le transport du dispositif d'irradiation par rayonnement, et
notifier l'utilisateur de l'assistance de transport du trajet de déplacement du dispositif d'irradiation par rayonnement jusqu'à la position cible sur la base de la position cible, de la position actuelle, et de la position de l'obstacle.

2. Procédé d'assistance de transport pour un dispositif d'irradiation par rayonnement selon la revendication 1, dans lequel la notification est démarrée dans un cas où le dispositif d'irradiation par rayonnement approche de la position cible à raison d'une distance prédéterminée, laquelle est plus courte qu'une distance jusqu'à la position cible au temps de réglage de la position cible, après le réglage de la position cible.

3. Procédé d'assistance de transport pour un dispositif d'irradiation par rayonnement selon la revendication 1, dans lequel la notification est démarrée dans un cas où le dispositif d'irradiation par rayonnement approche de l'obstacle à raison d'une distance prédéterminée, après le réglage de la position cible.

4. Procédé d'assistance de transport pour un dispositif d'irradiation par rayonnement selon l'une quelconque des revendications 1 à 3, dans lequel dans un cas où le dispositif d'irradiation par rayonnement est positionné sur la position cible, l'espace de travail est sécurisé sur au moins un côté droit du dispositif d'irradiation par rayonnement dans un cas où la position d'irradiation par rayonnement de la source de rayonnement est présente sur le côté droit du dispositif d'irradiation par rayonnement, et l'espace de travail est sécurisé sur au moins un côté gauche du dispositif d'irradiation par rayonnement dans un cas où la position d'irradiation par rayonnement de la source de rayonnement est présente sur le côté gauche du dispositif d'irradiation par rayonnement.

5. Procédé d'assistance de transport pour un dispositif d'irradiation par rayonnement selon l'une quelconque des revendications 1 à 4, dans lequel une position du support d'enregistrement d'image de rayonnement, laquelle est utilisée pour régler la position cible, est détectée sur la base d'une image, laquelle est reconstruite à partir d'images prises par une pluralité de caméras montées sur le dispositif d'irradiation par rayonnement et/ou d'une image prise par une seule caméra.

6. Procédé d'assistance de transport pour un dispositif d'irradiation par rayonnement selon l'une quelconque des revendications 1 à 5, dans lequel une position du support d'enregistrement d'image de rayonnement, laquelle est utilisée pour régler la position cible, est détectée sur la base d'un signal radio, lequel est transmis à partir du support d'enregistrement d'image de rayonnement.

7. Procédé d'assistance de transport pour un dispositif d'irradiation par rayonnement selon l'une quelconque des revendications 1 à 6, dans lequel une position du support d'enregistrement d'image de rayonnement, laquelle est utilisée pour régler la position cible, est acquise à partir d'un ordre pour prendre une image de rayonnement.

8. Procédé d'assistance de transport pour un dispositif d'irradiation par rayonnement selon l'une quelconque des revendications 1 à 7, dans lequel la position actuelle du dispositif d'irradiation par rayonnement est détectée sur la base d'une image, laquelle est reconstruite à partir d'images prises par une pluralité de caméras montées sur le dispositif d'irradiation par rayonnement et/ou d'une image prise par une seule caméra.

9. Procédé d'assistance de transport pour un dispositif d'irradiation par rayonnement selon l'une quelconque des revendications 1 à 8, dans lequel la position de l'obstacle est détectée sur la base d'une image, laquelle est reconstruite à partir d'images prises par une pluralité de caméras montées sur le dispositif d'irradiation par rayonnement et/ou d'une image prise par une seule caméra.

10. Procédé d'assistance de transport pour un dispositif d'irradiation par rayonnement selon l'une quelconque des revendications 1 à 9, dans lequel le réglage de la position cible est réalisé dans des conditions où la source de rayonnement peut être disposée sur la position d'irradiation par rayonnement, où une image de rayonnement peut être prise par rapport au support d'enregistrement d'image de rayonnement dans une plage pouvant être déplacée où la position de la source de rayonnement peut être modifiée par l'unité de tenue de source de rayonnement ; l'espace de travail peut être sécurisé autour du dispositif d'irradiation par rayonnement, et l'obstacle n'est pas présent dans une plage pouvant être déplacée de l'unité de tenue de source de rayonnement.

11. Procédé d'assistance de transport pour un dispositif d'irradiation par rayonnement selon l'une quelconque des revendications 1 à 9, dans lequel le réglage de la position cible est réalisé sous des conditions où la source de rayonnement peut être disposée sur la position d'irradiation par rayonnement, où une image de rayonnement peut être prise par rapport au support d'enregistrement d'image de rayonnement dans une plage pouvant être déplacée où la position de la source de rayonnement peut être modifiée par l'unité de tenue de source de rayonnement ; l'espace de travail peut être sécurisé autour du dispositif d'irradiation par rayonnement, et si oui ou non une image de rayonnement ne peut pas être prise en raison de la présence de l'obstacle dans un cas où l'obstacle est présent dans une plage pouvant être déplacée de l'unité de tenue de source de rayonnement.

12. Procédé d'assistance de transport pour un dispositif d'irradiation par rayonnement selon l'une quelconque des revendications 1 à 11, dans lequel la notification est réalisée à l'aide d'une image, laquelle est affichée sur des moyens d'affichage pour afficher une image.

13. Procédé d'assistance de transport pour un dispositif d'irradiation par rayonnement selon la revendication 12, dans lequel l'image, laquelle est affichée sur le moyen d'affichage comme notification, inclut un affichage, lequel montre un trajet jusqu'à la position cible à partir de la position actuelle du dispositif d'irradiation par rayonnement.

14. Procédé d'assistance de transport pour un dispositif d'irradiation par rayonnement selon la revendication 12 ou 13, dans lequel l'image, laquelle est affichée sur le moyen d'affichage comme notification, inclut un affichage, lequel montre un champ de vue sur un côté avant du dispositif d'irradiation par rayonnement dans une direction de transport.

15. Procédé d'assistance de transport pour un dispositif d'irradiation par rayonnement selon la revendication 12 ou 13, dans lequel l'image, laquelle est affichée sur le moyen d'affichage comme notification, inclut une image obtenue par la vue du dispositif d'irradiation par rayonnement par le dessus.

16. Procédé d'assistance de transport pour un dispositif d'irradiation par rayonnement selon l'une quelconque des revendications 1 à 11, dans lequel la notification est réalisée par une voix.

17. Procédé d'assistance de transport pour un dispositif d'irradiation par rayonnement selon la revendication 16, dans lequel la notification à l'aide de la voix est destinée à notifier l'utilisateur d'une direction de transport par rapport à la direction de transport du dispositif d'irradiation par rayonnement à un point dans le temps dans un cas où la notification est réalisée.

18. Procédé d'assistance de transport pour un dispositif d'irradiation par rayonnement selon l'une quelconque des revendications 1 à 17, dans lequel dans un cas où le dispositif d'irradiation par rayonnement s'écarte d'un trajet de déplacement approprié sur la base de la notification durant le transport du dispositif d'irradiation par rayonnement, une notification d'un avertissement indiquant un fait lié à l'écart du dispositif d'irradiation par rayonnement est générée.

19. Procédé d'assistance de transport pour un dispositif d'irradiation par rayonnement selon l'une quelconque des revendications 1 à 18, dans lequel dans un cas où le dispositif d'irradiation par rayonnement arrive sur la position cible, une notification à l'utilisateur de l'arrivée du dispositif d'irradiation par rayonnement est générée.

20. Dispositif d'assistance de transport pour un dispositif d'irradiation par rayonnement assistant un utilisateur pour transporter un dispositif d'irradiation par rayonnement (1), sur lequel sont montés une source de rayonnement (40), laquelle est configurée pour irradier avec un rayonnement un support d'enregistrement d'image de rayonnement (80), lequel est disposé sous un sujet (H) sur un lit, et une unité de tenue de source de rayonnement pouvant être déplacée (30), laquelle est configurée pour tenir la source de rayonnement (40) de manière à permettre de modifier la position de la source de rayonnement, dans lequel le dispositif d'irradiation par rayonnement peut être transporté par l'utilisateur jusqu'à une position cible, le dispositif d'assistance de transport comprenant :
un moyen de réglage de position cible pour régler la position cible du dispositif d'irradiation par rayonnement (1), où le support d'enregistrement d'image de rayonnement (80) se trouve dans une plage pouvant être déplacée de l'unité de tenue de source de rayonnement (30) de telle sorte que la position de la source de rayonnement (40) peut être modifiée par l'unité de tenue de source de rayonnement et que la source de rayonnement peut être disposée sur une position d'irradiation par rayonnement où une image de rayonnement peut être prise, et où un espace de travail, lequel est réglé sur une taille permettant à au moins une personne de passer facilement, peut être sécurisé autour du dispositif d'irradiation par rayonnement (1) ;
un moyen de détection de positon actuelle pour détecter une position actuelle du dispositif d'irradiation par rayonnement ;
un moyen de détection de position d'obstacle pour détecter une position d'un d'obstacle, laquelle obstrue le transport du dispositif d'irradiation par rayonnement, et
un moyen de notification pour notifier l'utilisateur de l'assistance de transport du trajet de déplacement du dispositif d'irradiation par rayonnement jusqu'à la position cible sur la base de la position cible, de la position actuelle, et de la position de l'obstacle.

21. Dispositif d'irradiation par rayonnement, sur lequel sont montés une source de rayonnement, laquelle est configurée pour irradier un support d'enregistrement d'image de rayonnement avec un rayonnement, et une unité de tenue de source de rayonnement pouvant être déplacée, laquelle est configurée pour tenir la source de rayonnement de manière à permettre de modifier la position de la source de rayonnement, dans lequel il est possible que l'utilisateur transporte le dispositif d'irradiation par rayonnement jusqu'à une position cible, le dispositif d'assistance de transport comprenant : le dispositif d'assistance de transport selon la revendication 20.
